# EUROPEAN PATENT APPLICATION

(11) **EP 4 467 542 A1**
(43) Date of publication of application: **27.11.2024**
(21) Application number: 23742841.2
(22) Date of filing: 16.01.2023
(51) Int. Cl.: C07D 471/04, C07D 453/02, A61K 31/519, A61P 35/00

(54) **CRYSTAL FORM OF ALLYL-CONTAINING METHYLPYRIDOPYRIMIDINE COMPOUND**

(30) Priority: 21.01.2022 CN 202210072234; 24.01.2022 CN 202210080325
(71) Applicant: Medshine Discovery Inc., Nanjing, Jiangsu 210032 (CN)
(72) Inventor: WU, Lifang, Shanghai 200131 (CN); SUN, Fei, Shanghai 200131 (CN); DING, Charles Z., Shanghai 200131 (CN)
(74) Representative: Zaboliene, Reda
(86) International application number: PCT/CN2023/072313
(87) International publication number: WO 2023/138526

(57) **Abstract**

Disclosed is a crystal form of an allyl-containing methylpyridopyrimidine compound, and specifically discloses a crystal form of a compound as shown in formula (I) and of a salt of the compound.

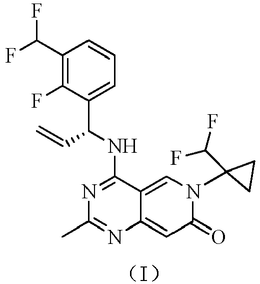

## Description

The present disclosure herein claims the following priority:
CN202210072234.1, application date: January 21, 2022; and
CN202210080325.X, application date: January 24, 2022.

### TECHNICAL FIELD

The present disclosure relates to a crystal form of an allyl-containing methylpyridopyrimidine compound, and specifically relates to a crystal form of a compound as shown in formula (I) and of a salt of the compound.

### BACKGROUND

Mutations in the Rat Sarcoma Virus Oncogene Homolog (RAS) protein occur in approximately 20% to 30% of cancer cases, and is the most common mutated oncogenes in pancreatic cancer, colorectal cancer, and non-small cell lung cancer. RAS proteins function as molecular switches, activated when bound to Guanosine-5'-triphosphate (GTP) and deactivated when bound to Guanosine-5'-diphosphate (GDP). Mutations in RAS proteins impair their ability to hydrolyze GTP with GTPases, causing the molecular switch to remain persistently active in the GTP-bound state. This drives unchecked downstream oncogenic signaling, such as through the RAS-Rapidly Accelerated Fibrosarcoma kinase (RAF)-MAPK/ERK Kinase (MEK)-Extracellular Signal-Regulated Kinase (ERK) pathway and the RAS-Phosphoinositide 3-Kinase (PI3K)-Phosphoinositide-Dependent Kinase-1 (PDK1)-Protein Kinase B (AKT) pathway, thereby promoting the survival and proliferation of cancer cells.

Son of Sevenless Homolog 1 (SOS1) protein is a Guanine Nucleotide Exchange Factor (GEF) that has been shown to catalyze the binding of Kirsten Rat Sarcoma Viral Oncogene Homolog (KRAS) to GTP, thereby facilitating the activation of KRAS. Consequently, the targeted inhibition of SOS1 protein and the SOS1-KRAS interaction, regardless of the specific KRAS mutation, emerges as a promising therapeutic target for cancer treatment. Furthermore, research has demonstrated that the combination of MEK inhibition can lead to profound pathway blockade and tumor regression in vivo, presenting a potential approach to treating a majority of KRAS-driven cancers.

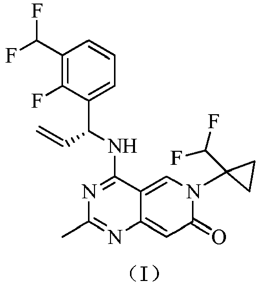

### SUMMARY

The present disclosure provides a crystal form A of a compound as shown in formula (I) 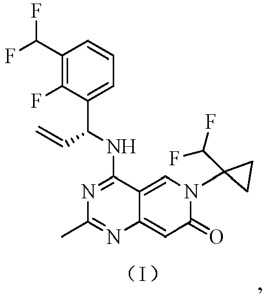 wherein an X-ray powder diffraction spectrum has diffraction peaks at the following 2θ angles: 9.41±0.20°, 19.23±0.20°, and 23.45±0.20°.

In some embodiments of the present disclosure, the above-mentioned crystal form A of the compound as shown in formula (I), wherein the X-ray powder diffraction spectrum has diffraction peaks at the following 2θ angles: 9.41±0.20°, 13.39±0.20°, 13.96±0.20°, 15.77±0.20°, and 19.23±0.20°.

In some embodiments of the present disclosure, the above-mentioned crystal form A of the compound as shown in formula (I), wherein the X-ray powder diffraction spectrum has diffraction peaks at the following 2θ angles: 9.41±0.20°, 13.39±0.20°, 13.96±0.20°, 15.77±0.20°, 19.23±0.20°, and 21.99±0.20°.

In some embodiments of the present disclosure, the above-mentioned crystal form A of the compound as shown in formula (I), wherein the X-ray powder diffraction spectrum has diffraction peaks at the following 2θ angles: 9.41±0.20°, 13.39±0.20°, 13.96±0.20°, 15.77±0.20°, 19.23±0.20°, 21.99±0.20°, and 23.45±0.20°.

In some embodiments of the present disclosure, the above-mentioned crystal form A of the compound as shown in formula (I), wherein the X-ray powder diffraction spectrum has diffraction peaks at the following 2θ angles: 9.41±0.20°, 13.39±0.20°, 13.96±0.20°, 15.77±0.20°, 19.23±0.20°, 21.99±0.20°, 23.45±0.20°, and 25.30±0.20°.

In some embodiments of the present disclosure, the above-mentioned crystal form A of the compound as shown in formula (I), wherein the X-ray powder diffraction spectrum has diffraction peaks at the following 2θ angles: 9.41±0.20°, 12.03±0.20°, 12.70±0.20°, 13.39±0.20°, 13.96±0.20°, 15.77±0.20°, 19.23±0.20°, 21.99±0.20°, 23.45±0.20°, and 25.30±0.20°.

In some embodiments of the present disclosure, the above-mentioned crystal form A of the compound as shown in formula (I), wherein the X-ray powder diffraction spectrum has diffraction peaks at the following 2θ angles: 9.41±0.20°, 10.13±0.20°, 12.03±0.20°, 12.70±0.20°, 13.39±0.20°, 13.96±0.20°, 14.98±0.20°, 15.77±0.20°, 18.86±0.20°, and 19.23±0.20°.

In some embodiments of the present disclosure, the above-mentioned crystal form A of the compound as shown in formula (I), wherein the X-ray powder diffraction spectrum has diffraction peaks at the following 2θ angles: 9.41±0.20°, 10.13±0.20°, 12.03±0.20°, 12.70±0.20°, 13.39±0.20°, 13.96±0.20°, 14.98±0.20°, 15.77±0.20°, 18.86±0.20°, 19.23±0.20°, and 21.99±0.20°.

In some embodiments of the present disclosure, the above-mentioned crystal form A of the compound as shown in formula (I), wherein the X-ray powder diffraction spectrum has diffraction peaks at the following 2θ angles: 9.41±0.20°, 10.13±0.20°, 12.03±0.20°, 12.70±0.20°, 13.39±0.20°, 13.96±0.20°, 14.67±0.20°, 14.98±0.20°, 15.77±0.20°, 16.12±0.20°, 16.67±0.20°, 17.94±0.20°, 18.28±0.20°, 18.86±0.20°, 19.23±0.20°, 19.69±0.20°, 20.12±0.20°, 21.99±0.20°, 22.35±0.20°, 22.74±0.20°, 23.45±0.20°, 23.71±0.20°, 24.35±0.20°, 25.30±0.20°, 25.80±0.20°, 26.15±0.20°, 26.51±0.20°, 28.38±0.20°, 29.94±0.20°, 30.34±0.20°, 30.64±0.20°, 32.02±0.20°, 32.87±0.20°, 37.62±0.20°, and 38.23±0.20°.

In some embodiments of the present disclosure, the above-mentioned crystal form A of the compound as shown in formula (I), wherein the X-ray powder diffraction spectrum has diffraction peaks at the following 2θ angles: 9.41°, 10.13°, 12.03°, 12.70°, 13.39°, 13.96°, 14.67°, 14.98°, 15.77°, 16.12°, 16.67°, 17.94°, 18.28°, 18.86°, 19.23°, 19.69°, 20.12°, 21.99°, 22.35°, 22.74°, 23.45°, 23.71°, 24.35°, 25.30°, 25.80°, 26.15°, 26.51°, 28.38°, 29.94°, 30.34°, 30.64°, 32.02°, 32.87°, 37.62°, and 38.23°.

In some embodiments of the present disclosure, the above-mentioned crystal form A of the compound as shown in formula (I), wherein the X-ray powder diffraction spectrum has diffraction peaks at the following 2θ angles: 9.41±0.20°, and/or 10.13±0.20°, and/or 12.03±0.20°, and/or 12.70±0.20°, and/or 13.39±0.20°, and/or 13.96±0.20°, and/or 14.67±0.20°, and/or 14.98±0.20°, and /or 15.77±0.20°, and/or 16.12±0.20°, and/or 16.67±0.20°, and/or 17.94±0.20°, and/or 18.28±0.20°, and/or 18.86±0.20°, and/or 19.23±0.20°, and/or 19.69±0.20°, and/or 20.12±0.20°, and/or 21.99±0.20°, and/or 22.35±0.20°, and/or 22.74±0.20°, and/or 23.45±0.20°, and/or 23.71±0.20°, and/or 24.35±0.20°, and/or 25.30±0.20°, and/or 25.80±0.20°, and/or 26.15±0.20°, and/or 26.51±0.20°, and/or 28.38±0.20°, and/or 29.94±0.20°, and/or 30.34±0.20°, and/or 30.64±0.20°, and/or 32.02±0.20°, and/or 32.87±0.20°, and /or 37.62±0.20°, and/or 38.23±0.20°.

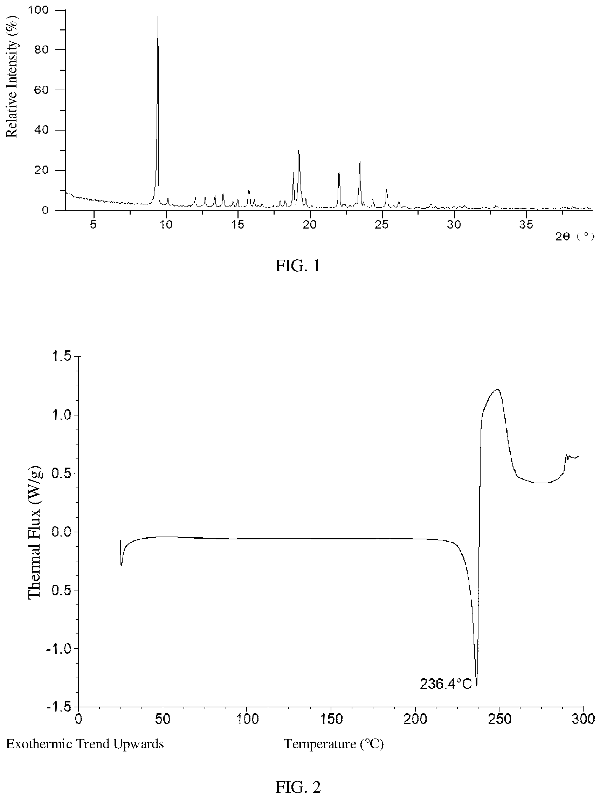

In some embodiments of the present disclosure, the X-ray Powder Diffraction (XRPD) spectrum of the above-mentioned crystal form A of the compound as shown in formula (I) is shown in FIG. 1.

In some embodiments of the present disclosure, the XRPD spectrum analysis data of Cu Kα radiation of the above-mentioned crystal form A of the compound as shown in formula (I) is shown in Table 1:

**Table 1 XRPD diffraction peak data of the crystal form A of the compound as shown in formula (I)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 9.41 | 100.0 | 19 | 22.35 | 1.9 |
| 2 | 10.13 | 4.1 | 20 | 22.74 | 1.3 |
| 3 | 12.03 | 5.1 | 21 | 23.45 | 25.3 |
| 4 | 12.70 | 4.9 | 22 | 23.71 | 3.2 |
| 5 | 13.39 | 6.0 | 23 | 24.35 | 4.9 |
| 6 | 13.96 | 7.1 | 24 | 25.30 | 10.5 |
| 7 | 14.67 | 2.8 | 25 | 25.80 | 1.3 |
| 8 | 14.98 | 4.0 | 26 | 26.15 | 3.6 |
| 9 | 15.77 | 9.3 | 27 | 26.51 | 1.0 |
| 10 | 16.12 | 4.2 | 28 | 28.38 | 2.2 |
| 11 | 16.67 | 2.1 | 29 | 29.94 | 1.1 |
| 12 | 17.94 | 3.5 | 30 | 30.34 | 1.1 |
| 13 | 18.28 | 3.6 | 31 | 30.64 | 1.6 |
| 14 | 18.86 | 19.9 | 32 | 32.02 | 0.9 |
| 15 | 19.23 | 32.5 | 33 | 32.87 | 2.0 |
| 16 | 19.69 | 4.9 | 34 | 37.62 | 0.7 |
| 17 | 20.12 | 1.0 | 35 | 38.23 | 0.8 |
| 18 | 21.99 | 19.6 | / | / | / |

In some embodiments of the present disclosure, the above-mentioned crystal form A of the compound as shown in formula (I), wherein the X-ray powder diffraction spectrum is shown in FIG. 1.

In some embodiments of the present disclosure, the above-mentioned crystal form A of the compound as shown in formula (I), wherein a Differential Scanning Calorimetry (DSC) Curve has an endothermic peak at 236.4±3°C.

In some embodiments of the present disclosure, the above-mentioned crystal form A of the compound as shown in formula (I), wherein the differential scanning calorimetry curve spectrum is shown in FIG. 2.

In some embodiments of the present disclosure, the above-mentioned crystal form A of the compound as shown in formula (I), wherein a thermogravimetric analysis curve has a weight loss of 1.24% at 150.0±3°C.

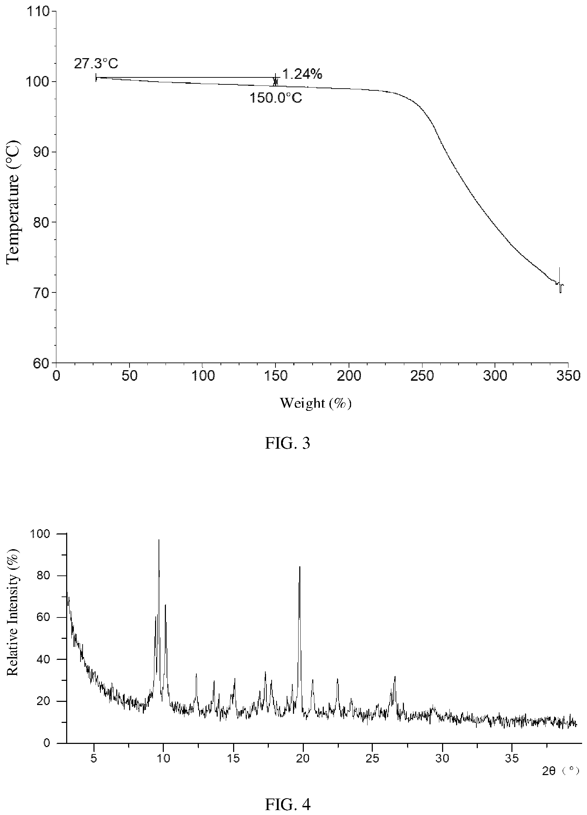

In some embodiments of the present disclosure, the above-mentioned crystal form A of the compound as shown in formula (I), wherein the thermogravimetric analysis curve spectrum is shown in FIG. 3. According to the Thermogravimetric Analysis (TGA) results, it can be seen that the above-mentioned crystal form A of the compound as shown in formula (I) is an anhydrous crystal form.

The present disclosure provides a crystal form B of a compound as shown in formula (I) 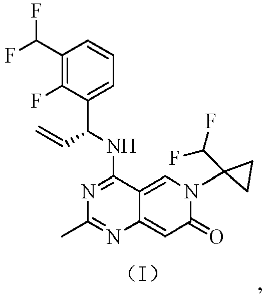 wherein an X-ray powder diffraction spectrum has diffraction peaks at the following 2θ angles: 9.66±0.20°, 10.15±0.20°, and 19.73±0.20°.

In some embodiments of the present disclosure, the above-mentioned crystal form B of the compound as shown in formula (I), wherein the X-ray powder diffraction spectrum has diffraction peaks at the following 2θ angles: 9.66±0.20°, 10.15±0.20°, 17.32±0.20°, 17.74±0.20°, 19.73±0.20°, 22.46±0.20°, and 26.59±0.20°.

In some embodiments of the present disclosure, the above-mentioned crystal form B of the compound as shown in formula (I), wherein the X-ray powder diffraction spectrum has diffraction peaks at the following 2θ angles: 9.40±0.20°, 9.66±0.20°, 10.15±0.20°, 12.34±0.20°, 13.59±0.20°, 15.10±0.20°, 17.32±0.20°, 17.74±0.20°, 19.73±0.20°, 20.69±0.20°, 22.46±0.20°, and 26.59±0.20°.

In some embodiments of the present disclosure, the above-mentioned crystal form B of the compound as shown in formula (I), wherein the X-ray powder diffraction spectrum has diffraction peaks at the following 2θ angles: 9.40°, 9.66°, 10.15°, 12.34°, 13.59°, 15.10°, 17.32°, 17.74°, 19.73°, 20.69°, 22.46°, and 26.59°.

In some embodiments of the present disclosure, the above-mentioned crystal form B of the compound as shown in formula (I), wherein the X-ray powder diffraction spectrum has diffraction peaks at the following 2θ angles: 9.66±0.20°, and/or 9.40±0.20°, and/or 10.15±0.20°, and/or 12.34±0.20°, and/or 13.59±0.20°, and/or 15.10±0.20°, and/or 17.32±0.20°, and/or 17.74±0.20°, and /or 19.73±0.20°, and/or 20.69±0.20°, and/or 22.46±0.20°, and/or 26.59±0.20°.

In some embodiments of the present disclosure, the XRPD spectrum of the above-mentioned crystal form B of the compound as shown in formula (I) is shown in FIG. 4.

In some embodiments of the present disclosure, the XRPD spectrum analysis data of Cu Kα radiation of the above-mentioned crystal form B of the compound as shown in formula (I) is shown in Table 2:

**Table 2 XRPD diffraction peak data of the crystal form B of the compound as shown in formula (I)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 9.40 | 51.4 | 7 | 17.32 | 23.1 |
| 2 | 9.66 | 100.0 | 8 | 17.74 | 18.7 |
| 3 | 10.15 | 64.6 | 9 | 19.73 | 89.6 |
| 4 | 12.34 | 18.3 | 10 | 20.69 | 18.6 |
| 5 | 13.59 | 14.1 | 11 | 22.46 | 21.1 |
| 6 | 15.10 | 18.0 | 12 | 26.59 | 22.0 |

In some embodiments of the present disclosure, a Differential Scanning Calorimetry (DSC) Curve of the above-mentioned crystal form B of the compound as shown in formula (I) has an endothermic peak at 240.2±3°C.

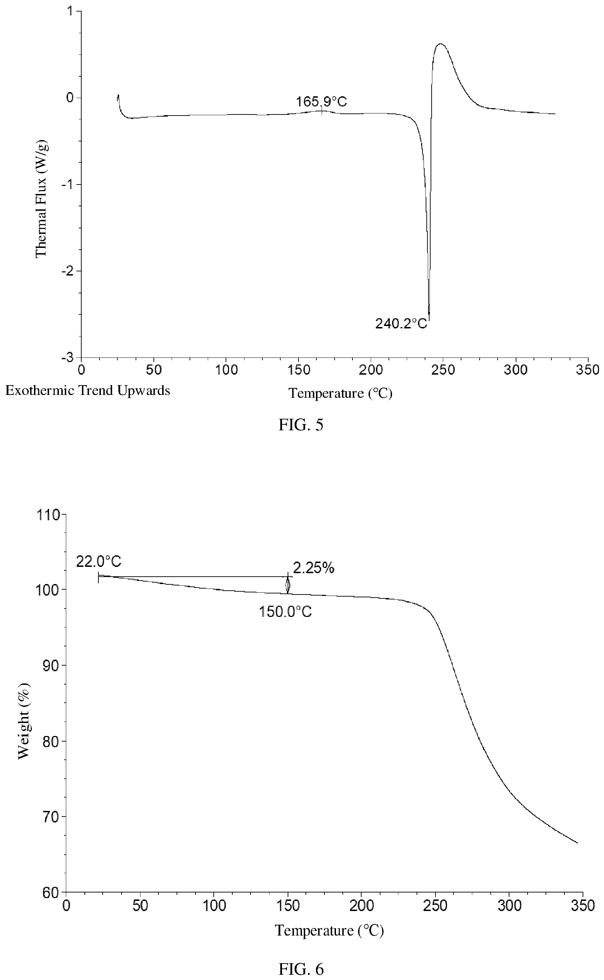

In some embodiments of the present disclosure, the DSC spectrum of the above-mentioned crystal form B of the compound as shown in formula (I) is shown in FIG. 5.

In some embodiments of the present disclosure, a Thermogravimetric Analysis (TGA) curve of the above-mentioned crystal form B of the compound as shown in formula (I) has a weight loss of 2.25% at 150.0±3°C. According to the TGA results, it can be seen that the above-mentioned crystal form B of the compound as shown in formula (I) is an anhydrous crystal form.

In some embodiments of the present disclosure, the TGA spectrum of the above-mentioned crystal form B of the compound as shown in formula (I) is shown in FIG. 6.

The present disclosure provides a crystal form C of a compound as shown in formula (I) 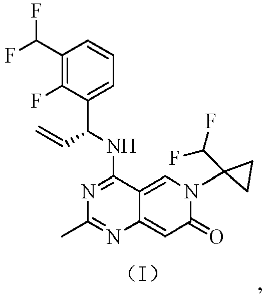 wherein an X-ray powder diffraction spectrum has diffraction peaks at the following 2θ angles: 9.35±0.20°, 19.08±0.20°, and 22.91±0.20°.

In some embodiments of the present disclosure, the above-mentioned crystal form C of the compound as shown in formula (I), wherein the X-ray powder diffraction spectrum has diffraction peaks at the following 2θ angles: 9.35±0.20°, 10.08±0.20°, 15.97±0.20°, 18.68±0.20°, 19.08±0.20°, 21.86±0.20°, 22.91±0.20°, and 25.45±0.20°.

In some embodiments of the present disclosure, the above-mentioned crystal form C of the compound as shown in formula (I), wherein the X-ray powder diffraction spectrum has diffraction peaks at the following 2θ angles: 9.35±0.20°, 10.08±0.20°, 10.39±0.20°, 11.24±0.20°, 11.94±0.20°, 13.50±0.20°, 15.97±0.20°, 17.50±0.20°, 18.68±0.20°, 19.08±0.20°, 20.18±0.20°, 21.86±0.20°, 22.91±0.20°, 23.81±0.20°, 25.45±0.20°, 26.39±0.20°, 27.40±0.20°, 28.49±0.20°, 29.45±0.20°, 30.43±0.20°, and 32.31±0.20°.

In some embodiments of the present disclosure, the above-mentioned crystal form C of the compound as shown in formula (I), wherein the X-ray powder diffraction spectrum has diffraction peaks at the following 2θ angles: 9.35°, 10.08°, 10.39°, 11.24°, 11.94°, 13.50°, 15.97°, 17.50°, 18.68°, 19.08°, 20.18°, 21.86°, 22.91°, 23.81°, 25.45°, 26.39°, 27.40°, 28.49°, 29.45°, 30.43°, and 32.31°.

In some embodiments of the present disclosure, the above-mentioned crystal form C of the compound as shown in formula (I), wherein the X-ray powder diffraction spectrum has diffraction peaks at the following 2θ angles: 9.35±0.20°, and/or 10.08±0.20°, and/or 10.39±0.20°, and/or 11.24±0.20°, and/or 11.94±0.20°, and/or 13.50±0.20°, and/or 15.97±0.20°, and/or 17.50±0.20°, and/or 18.68±0.20°, and/or 19.08±0.20°, and/or 20.18±0.20°, and/or 21.86±0.20°, and/or 22.91±0.20°, and/or 23.81±0.20°, and/or 25.45±0.20°, and/or 26.39±0.20°, and/or 27.40±0.20°, and/or 28.49±0.20°, and/or 29.45±0.20°, and/or 30.43±0.20°, and/or 32.31±0.20°.

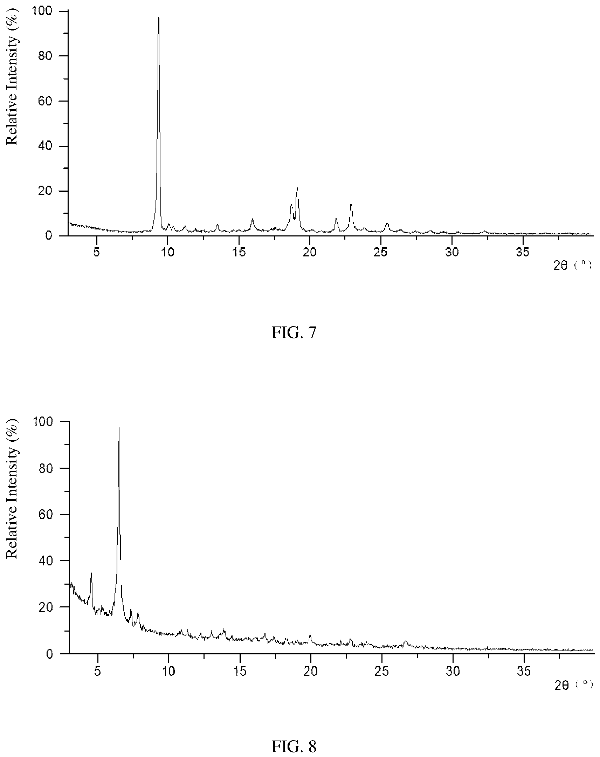

In some embodiments of the present disclosure, the XRPD spectrum of the above-mentioned crystal form C of the compound as shown in formula (I) is shown in FIG. 7.

In some embodiments of the present disclosure, the XRPD spectrum analysis data of Cu Kα radiation of the above-mentioned crystal form C of the compound as shown in formula (I) is shown in Table 3:

**Table 3 XRPD diffraction peak data of the crystal form C of the compound as shown in formula (I)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 9.35 | 100.0 | 12 | 21.86 | 6.2 |
| 2 | 10.08 | 3.6 | 13 | 22.91 | 13.5 |
| 3 | 10.39 | 2.7 | 14 | 23.81 | 2.6 |
| 4 | 11.24 | 3.1 | 15 | 25.45 | 4.5 |
| 5 | 11.94 | 1.4 | 16 | 26.39 | 1.7 |
| 6 | 13.50 | 3.6 | 17 | 27.40 | 1.0 |
| 7 | 15.97 | 6.0 | 18 | 28.49 | 1.3 |
| 8 | 17.50 | 2.0 | 19 | 29.45 | 0.7 |
| 9 | 18.68 | 12.7 | 20 | 30.43 | 0.7 |
| 10 | 19.08 | 20.1 | 21 | 32.31 | 1.1 |
| 11 | 20.18 | 1.4 | / | / | / |

In some embodiments of the present disclosure, the above-mentioned crystal form C of the compound as shown in formula (I) is obtained by heating the crystal form B of the compound as shown in formula (I) to 180°C in a nitrogen atmosphere. The above-mentioned crystal form C of the compound as shown in formula (I) is converted into the crystal form A of the compound as shown in formula (I) after cooling to 30°C in a nitrogen atmosphere.

The present disclosure provides a crystal form D of a compound as shown in formula (II) 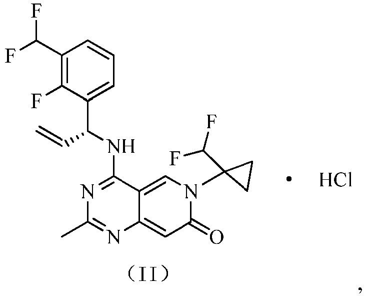 wherein an X-ray powder diffraction spectrum has diffraction peaks at the following 2θ angles: 4.56°, 6.50°, 7.83°, 13.91°, 16.69°, 18.23°, 19.94°, 22.80°, and 26.64°.

In some embodiments of the present disclosure, the above-mentioned crystal form D of the compound as shown in formula (II), wherein the X-ray powder diffraction spectrum has diffraction peaks at the following 2θ angles: 4.56±0.20°, 6.50±0.20°, 7.83±0.20°, 13.91±0.20°, 16.69±0.20°, 18.23±0.20°, 19.94±0.20°, 22.80±0.20°, and 26.64±0.20°.

In some embodiments of the present disclosure, the above-mentioned crystal form D of the compound as shown in formula (II), wherein the X-ray powder diffraction spectrum has diffraction peaks at the following 2θ angles: 6.50±0.20°, and/or 4.56±0.20°, and/or 7.83±0.20°, and/or 13.91±0.20°, and/or 16.69±0.20°, and/or 18.23±0.20°, and/or 19.94±0.20°, and/or 22.80±0.20°, and/or 26.64±0.20°.

In some embodiments of the present disclosure, the XRPD spectrum of the above-mentioned crystal form D of the compound as shown in formula (II) is shown in FIG. 8.

In some embodiments of the present disclosure, the XRPD spectrum analysis data of Cu Kα radiation of the above-mentioned crystal form D of the compound as shown in formula (II) is shown in Table 4:

**Table 4 XRPD diffraction peak data of the crystal form D of the compound as shown in formula (II)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 4.56 | 18.2 | 6 | 18.23 | 2.2 |
| 2 | 6.50 | 100.0 | 7 | 19.94 | 5.4 |
| 3 | 7.83 | 6.3 | 8 | 22.80 | 3.2 |
| 4 | 13.91 | 4.1 | 9 | 26.64 | 3.3 |
| 5 | 16.69 | 3.2 | / | / | / |

In some embodiments of the present disclosure, a Differential Scanning Calorimetry (DSC) Curve of the above-mentioned crystal form D of the compound as shown in formula (II) has an endothermic peak at 102.0±3°C.

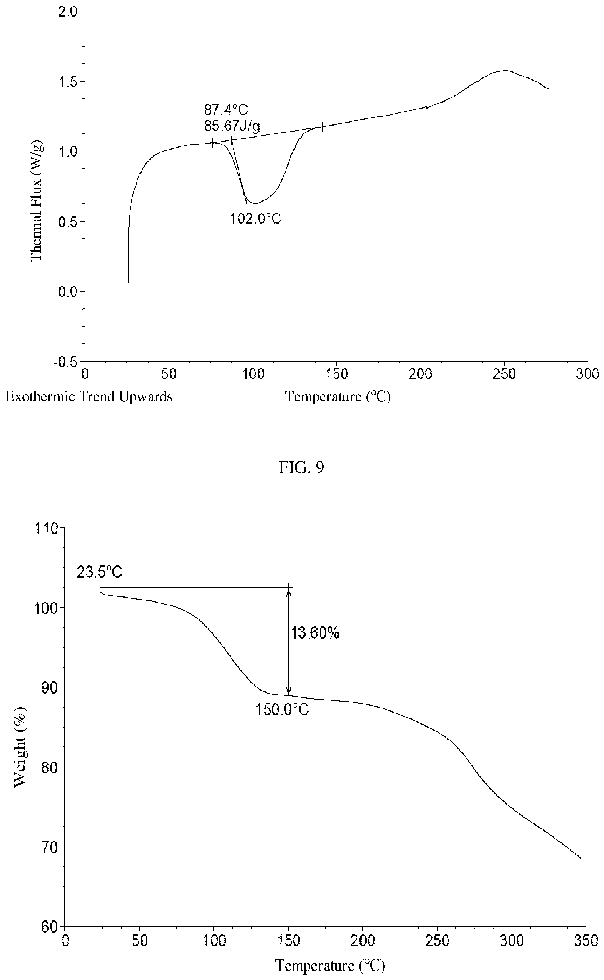

In some embodiments of the present disclosure, the DSC spectrum of the above-mentioned crystal form D of the compound as shown in formula (II) is shown in FIG. 9.

In some embodiments of the present disclosure, a Thermogravimetric Analysis (TGA) curve of the above-mentioned crystal form D of the compound as shown in formula (II) has a weight loss of 13.60% at 150.0±3°C.

In some embodiments of the present disclosure, the TGA spectrum of the above-mentioned crystal form D of the compound as shown in formula (II) is shown in FIG. 10.

In some embodiments of the present disclosure, the above-mentioned crystal form D of the compound as shown in formula (II) is prepared from the crystal form A of the compound as shown in formula (I) and hydrochloric acid in isopropyl alcohol. The Ultra Performance Liquid Chromatography (UPLC)/Ion Chromatography (IC) results show that the acid-base molar ratio is 1.0.

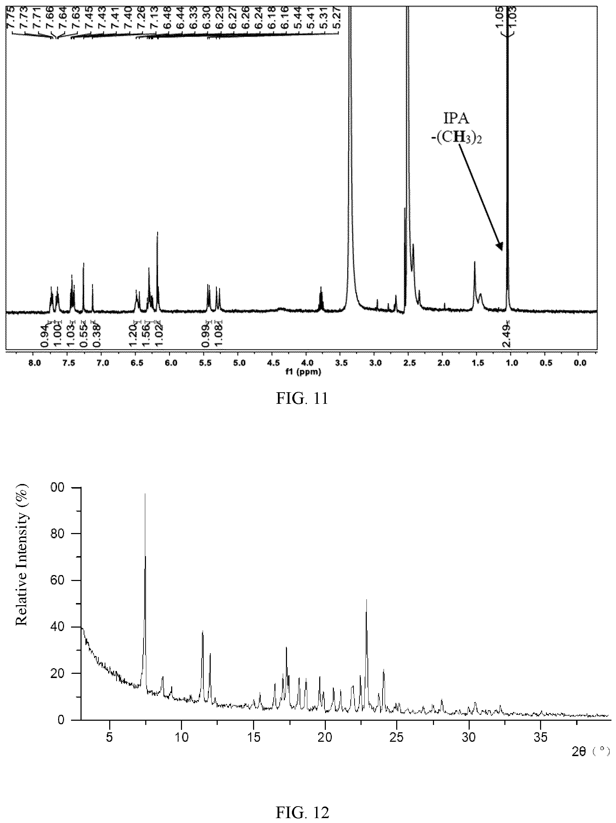

In some embodiments of the present disclosure, the ¹H Nuclear Magnetic Resonance (NMR) spectrum of the above-mentioned crystal form D of the compound as shown in formula (II) is shown in FIG. 11.

The present disclosure provides a crystal form E of a compound as shown in formula (III) 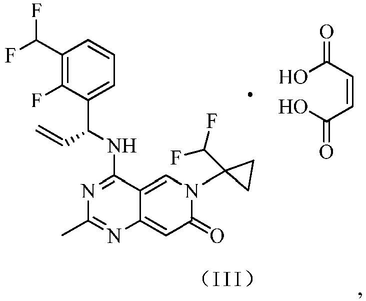 wherein an X-ray powder diffraction spectrum has diffraction peaks at the following 2θ angles: 7.45±0.20°, 11.45±0.20°, and 22.89±0.20°.

In some embodiments of the present disclosure, the above-mentioned crystal form E of the compound as shown in formula (III), wherein the X-ray powder diffraction spectrum has diffraction peaks at the following 2θ angles: 7.45±0.20°, 11.45±0.20°, 11.97±0.20°, 17.29±0.20°, 18.65±0.20°, 22.46±0.20°, 22.89±0.20°, and 24.08±0.20°.

In some embodiments of the present disclosure, the above-mentioned crystal form E of the compound as shown in formula (III), wherein the X-ray powder diffraction spectrum has diffraction peaks at the following 2θ angles: 7.45±0.20°, 8.67±0.20°, 11.45±0.20°, 11.97±0.20°, 15.43±0.20°, 16.48±0.20°, 17.05±0.20°, 17.29±0.20°, 18.16±0.20°, 18.65±0.20°, 19.61±0.20°, 19.88±0.20°, 20.60±0.20°, 21.07±0.20°, 21.97±0.20°, 22.46±0.20°, 22.89±0.20°, 23.73±0.20°, 24.08±0.20°, 25.14±0.20°, 26.84±0.20°, 27.49±0.20°, 28.12±0.20°, 29.99±0.20°, 30.43±0.20°, and 32.20±0.20°.

In some embodiments of the present disclosure, the above-mentioned crystal form E of the compound as shown in formula (III), wherein the X-ray powder diffraction spectrum has diffraction peaks at the following 2θ angles: 7.45°, 8.67°, 11.45°, 11.97°, 15.43°, 16.48°, 17.05°, 17.29°, 18.16°, 18.65°, 19.61°, 19.88°, 20.60°, 21.07°, 21.97°, 22.46°, 22.89°, 23.73°, 24.08°, 25.14°, 26.84°, 27.49°, 28.12°, 29.99°, 30.43°, and 32.20°.

In some embodiments of the present disclosure, the above-mentioned crystal form E of the compound as shown in formula (III), wherein the X-ray powder diffraction spectrum has diffraction peaks at the following 2θ angles: 7.45±0.20°, and/or 8.67±0.20°, and/or 11.45±0.20°, and/or 11.97±0.20°, and/or 15.43±0.20°, and/or 16.48±0.20°, and/or 17.05±0.20°, and/or 17.29±0.20°, and/or 18.16±0.20°, and/or 18.65±0.20°, and/or 19.61±0.20°, and/or 19.88±0.20°, and/or 20.60±0.20°, and/or 21.07±0.20°, and/or 21.97±0.20°, and/or 22.46±0.20°, and/or 22.89±0.20°, and/or 23.73±0.20°, and/or 24.08±0.20°, and/or 25.14±0.20°, and/or 26.84±0.20°, and/or 27.49±0.20°, and/or 28.12±0.20°, and/or 29.99±0.20°, and/or 30.43±0.20°, and/or 32.20±0.20°.

In some embodiments of the present disclosure, the XRPD spectrum of the above-mentioned crystal form E of the compound as shown in formula (III) is shown in FIG. 12.

In some embodiments of the present disclosure, the XRPD spectrum analysis data of Cu Kα radiation of the above-mentioned crystal form E of the compound as shown in formula (III) is shown in Table 5:

**Table 5 XRPD diffraction peak data of the crystal form E of the compound as shown in formula (III)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 7.45 | 100.0 | 14 | 21.07 | 10.7 |
| 2 | 8.67 | 10.3 | 15 | 21.97 | 13.2 |
| 3 | 11.45 | 40.6 | 16 | 22.46 | 18.4 |
| 4 | 11.97 | 26.9 | 17 | 22.89 | 58.6 |
| 5 | 15.43 | 8.2 | 18 | 23.73 | 9.6 |
| 6 | 16.48 | 13.9 | 19 | 24.08 | 23.0 |
| 7 | 17.05 | 17.7 | 20 | 25.14 | 4.8 |
| 8 | 17.29 | 32.7 | 21 | 26.84 | 3.0 |
| 9 | 18.16 | 17.0 | 22 | 27.49 | 4.0 |
| 10 | 18.65 | 17.3 | 23 | 28.12 | 7.7 |
| 11 | 19.61 | 17.0 | 24 | 29.99 | 3.2 |
| 12 | 19.88 | 10.2 | 25 | 30.43 | 6.3 |
| 13 | 20.60 | 12.3 | 26 | 32.20 | 3.9 |

In some embodiments of the present disclosure, a Differential Scanning Calorimetry (DSC) Curve of the above-mentioned crystal form E of the compound as shown in formula (III) has an endothermic peak at 205.1±3°C.

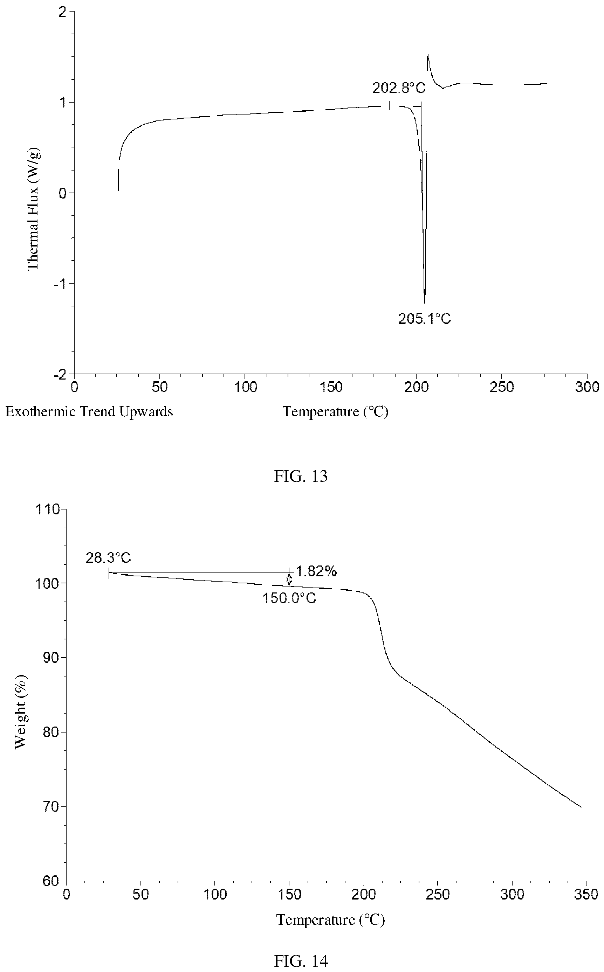

In some embodiments of the present disclosure, the DSC spectrum of the above-mentioned crystal form E of the compound as shown in formula (III) is shown in FIG. 13.

In some embodiments of the present disclosure, a Thermogravimetric Analysis (TGA) curve of the above-mentioned crystal form E of the compound as shown in formula (III) has a weight loss of 1.82% at 150.0±3°C.

In some embodiments of the present disclosure, the TGA spectrum of the above-mentioned crystal form E of the compound as shown in formula (III) is shown in FIG. 14.

In some embodiments of the present disclosure, the above-mentioned crystal form E of the compound as shown in formula (III) is prepared from the crystal form A of the compound as shown in formula (I) and maleic acid in solvent such as isopropyl alcohol, isopropyl acetate, and 2-methyltetrahydrofuran. The ¹H NMR results show that the molar ratio of maleic acid to the compound (free base) as shown in formula (I) in the above-mentioned crystal form E of the compound as shown in formula (III) is 1.0.

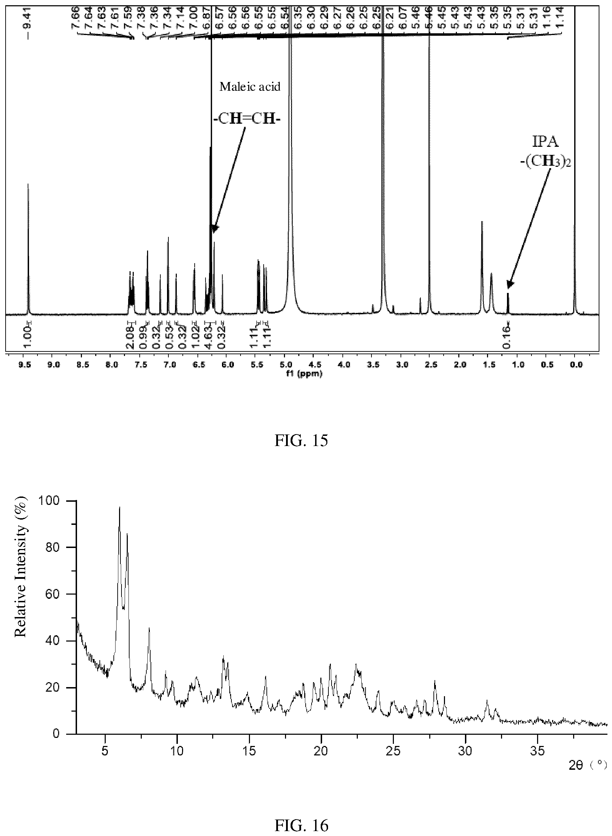

In some embodiments of the present disclosure, the ¹H NMR spectrum of the above-mentioned crystal form E of the compound as shown in formula (III) is shown in FIG. 15.

The present disclosure provides a crystal form F of a compound as shown in formula (IV) 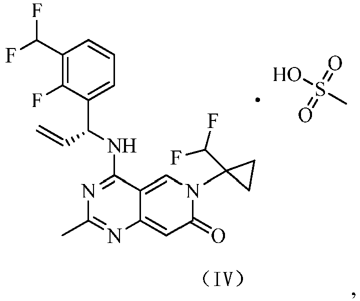 wherein an X-ray powder diffraction spectrum has diffraction peaks at the following 2θ angles: 6.01±0.20°, 6.56±0.20°, and 8.06±0.20°.

In some embodiments of the present disclosure, the above-mentioned crystal form F of the compound as shown in formula (IV), wherein the X-ray powder diffraction spectrum has diffraction peaks at the following 2θ angles: 6.01±0.20°, 6.56±0.20°, 8.06±0.20°, 13.20±0.20°, 16.14±0.20°, 20.61±0.20°, 22.40±0.20°, and 27.87±0.20°.

In some embodiments of the present disclosure, the above-mentioned crystal form F of the compound as shown in formula (IV), wherein the X-ray powder diffraction spectrum has diffraction peaks at the following 2θ angles: 6.01±0.20°, 6.56±0.20°, 8.06±0.20°, 9.22±0.20°, 9.70±0.20°, 11.34±0.20°, 13.20±0.20°, 13.53±0.20°, 14.87±0.20°, 16.14±0.20°, 17.08±0.20°, 18.44±0.20°, 18.76±0.20°, 19.49±0.20°, 20.00±0.20°, 20.61±0.20°, 21.00±0.20°, 22.40±0.20°, 23.93±0.20°, 24.89±0.20°, 26.65±0.20°, 27.18±0.20°, 27.87±0.20°, 28.57±0.20°, 31.50±0.20°, and 32.07±0.20°.

In some embodiments of the present disclosure, the above-mentioned crystal form F of the compound as shown in formula (IV), wherein the X-ray powder diffraction spectrum has diffraction peaks at the following 2θ angles: 6.01°, 6.56°, 8.06°, 9.22°, 9.70°, 11.34°, 13.20°, 13.53°, 14.87°, 16.14°, 17.08°, 18.44°, 18.76°, 19.49°, 20.00°, 20.61°, 21.00°, 22.40°, 23.93°, 24.89°, 26.65°, 27.18°, 27.87°, 28.57°, 31.50°, and 32.07°.

In some embodiments of the present disclosure, the above-mentioned crystal form F of the compound as shown in formula (IV), wherein the X-ray powder diffraction spectrum has diffraction peaks at the following 2θ angles: 6.01°, and/or 6.56°, and/or 8.06°, and/or 9.22°, and/or 9.70°, and/or 11.34°, and/or 13.20°, and/or 13.53°, and/or 14.87°, and/or 16.14°, and/or 17.08°, and/or 18.44°, and/or 18.76°, and/or 19.49°, and/or 20.00°, and/or 20.61°, and/or 21.00°, and/or 22.40°, and/or 23.93°, and/or 24.89°, and/or 26.65°, and/or 27.18°, and/or 27.87°, and/or 28.57°, and/or 31.50°, and/or 32.07°.

In some embodiments of the present disclosure, the XRPD spectrum of the above-mentioned crystal form F of the compound as shown in formula (IV) is shown in FIG. 16.

In some embodiments of the present disclosure, the XRPD spectrum analysis data of Cu Kα radiation of the above-mentioned crystal form F of the compound as shown in formula (IV) is shown in Table 6:

**Table 6 XRPD diffraction peak data of the crystal form F of the compound as shown in formula (IV)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 6.01 | 100.0 | 14 | 19.49 | 17.9 |
| 2 | 6.56 | 84.5 | 15 | 20.00 | 18.9 |
| 3 | 8.06 | 36.8 | 16 | 20.61 | 29.2 |
| 4 | 9.22 | 12.7 | 17 | 21.00 | 22.5 |
| 5 | 9.70 | 9.6 | 18 | 22.40 | 30.1 |
| 6 | 11.34 | 15.6 | 19 | 23.93 | 14.5 |
| 7 | 13.20 | 26.2 | 20 | 24.89 | 8.3 |
| 8 | 13.53 | 26.3 | 21 | 26.65 | 8.8 |
| 9 | 14.87 | 7.9 | 22 | 27.18 | 11.2 |
| 10 | 16.14 | 20.4 | 23 | 27.87 | 22.4 |
| 11 | 17.08 | 5.8 | 24 | 28.57 | 10.9 |
| 12 | 18.44 | 11.0 | 25 | 31.50 | 11.4 |
| 13 | 18.76 | 16.5 | 26 | 32.07 | 7.2 |

In some embodiments of the present disclosure, a Differential Scanning Calorimetry (DSC) Curve of the above-mentioned crystal form F of the compound as shown in formula (IV) has an endothermic peak at 95.2±3°C.

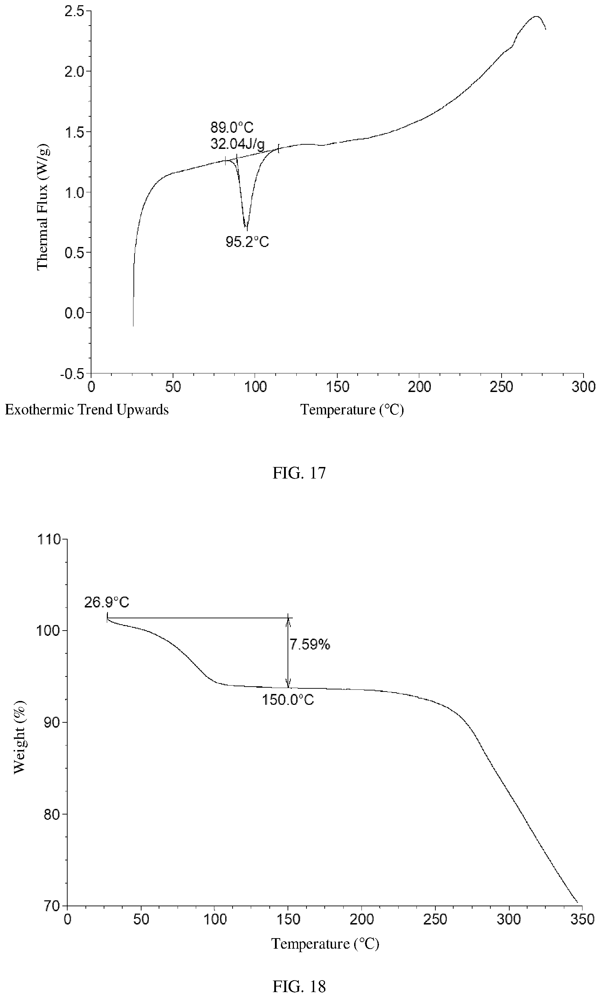

In some embodiments of the present disclosure, the DSC spectrum of the above-mentioned crystal form F of the compound as shown in formula (IV) is shown in FIG. 17.

In some embodiments of the present disclosure, a Thermogravimetric Analysis (TGA) curve of the above-mentioned crystal form F of the compound as shown in formula (IV) has a weight loss of 7.6% at 150.0±3°C.

In some embodiments of the present disclosure, the TGA spectrum of the above-mentioned crystal form F of the compound as shown in formula (IV) is shown in FIG. 18.

In some embodiments of the present disclosure, the above-mentioned crystal form F of the compound as shown in formula (IV) is prepared from the crystal form A of the compound as shown in formula (I) and methanesulfonic acid in isopropyl acetate. The ¹H NMR results show that the molar ratio of methanesulfonic acid to the compound (free base) as shown in formula (I) in the above-mentioned crystal form F of the compound as shown in formula (IV) is 0.9.

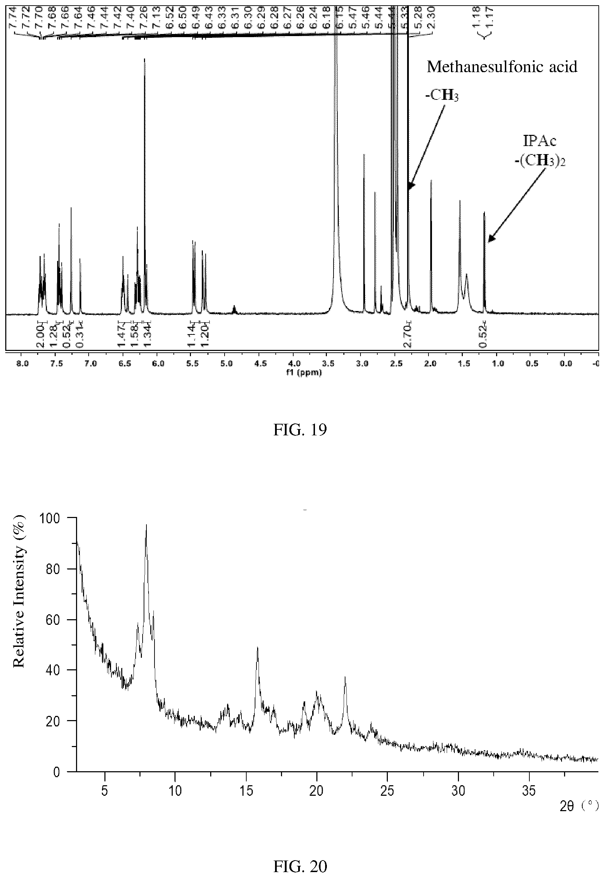

In some embodiments of the present disclosure, the ¹H NMR spectrum of the above-mentioned crystal form F of the compound as shown in formula (IV) is shown in FIG. 19.

The present disclosure provides a crystal form G of a compound as shown in formula (IV) 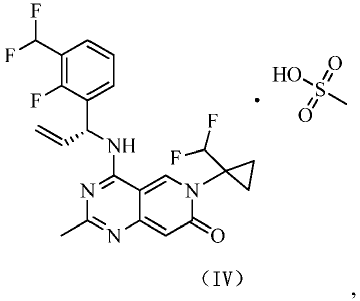 wherein an X-ray powder diffraction spectrum has diffraction peaks at the following 2θ angles: 7.32°, 7.91°, 8.48°, 13.48°, 15.81°, 19.08°, 19.89°, 22.01°, and 23.81°.

In some embodiments of the present disclosure, the above-mentioned crystal form G of the compound as shown in formula (IV), wherein the X-ray powder diffraction spectrum has diffraction peaks at the following 2θ angles: 7.32±0.20°, 7.91±0.20°, 8.48±0.20°, 13.48±0.20°, 15.81±0.20°, 19.08±0.20°, 19.89±0.20°, 22.01±0.20°, and 23.81±0.20°.

In some embodiments of the present disclosure, the above-mentioned crystal form G of the compound as shown in formula (IV), wherein the X-ray powder diffraction spectrum has diffraction peaks at the following 2θ angles: 7.32±0.20°, and/or 7.91±0.20°, and/or 8.48±0.20°, and/or 13.48±0.20°, and/or 15.81±0.20°, and/or 19.08±0.20°, and/or 19.89±0.20°, and/or 22.01±0.20°, and/or 23.81±0.20°.

In some embodiments of the present disclosure, the XRPD spectrum of the above-mentioned crystal form G of the compound as shown in formula (IV) is shown in FIG. 20.

In some embodiments of the present disclosure, the XRPD spectrum analysis data of Cu Kα radiation of the above-mentioned crystal form G of the compound as shown in formula (IV) is shown in Table 7:

**Table 7 XRPD diffraction peak data of the crystal form G of the compound as shown in formula (IV)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 7.32 | 40.5 | 6 | 19.08 | 15.9 |
| 2 | 7.91 | 100.0 | 7 | 19.89 | 18.2 |
| 3 | 8.48 | 52.2 | 8 | 22.01 | 34.3 |
| 4 | 13.48 | 7.7 | 9 | 23.81 | 7.4 |
| 5 | 15.81 | 47.7 | | | |

In some embodiments of the present disclosure, a Differential Scanning Calorimetry (DSC) Curve of the above-mentioned crystal form G of the compound as shown in formula (IV) has an endothermic peak at 106.3±3°C and 184.7±3°C.

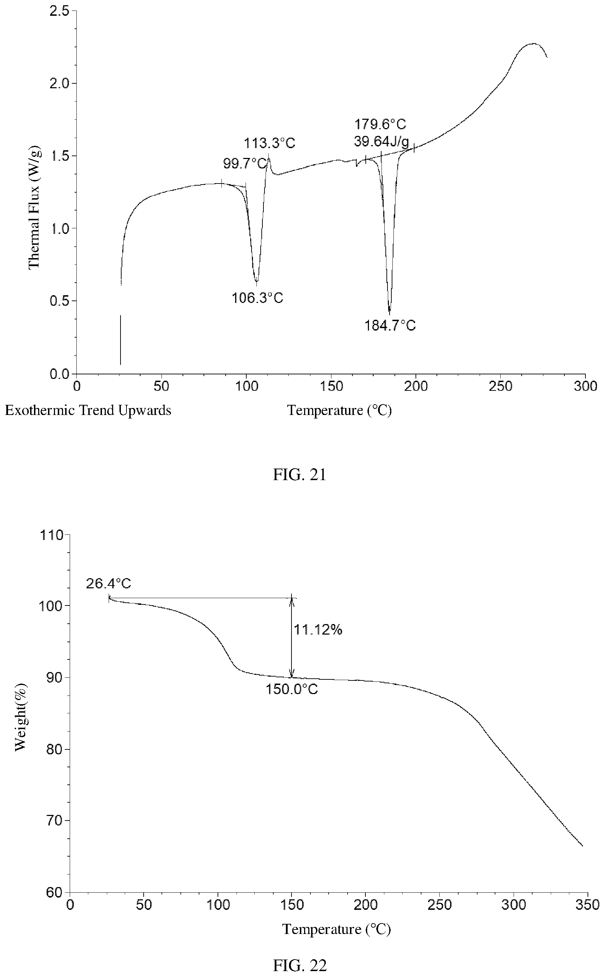

In some embodiments of the present disclosure, the DSC spectrum of the above-mentioned crystal form G of the compound as shown in formula (IV) is shown in FIG. 21.

In some embodiments of the present disclosure, a Thermogravimetric Analysis (TGA) curve of the above-mentioned crystal form G of the compound as shown in formula (IV) has a weight loss of 11.1% at 150.0±3°C.

In some embodiments of the present disclosure, the TGA spectrum of the above-mentioned crystal form G of the compound as shown in formula (IV) is shown in FIG. 22.

In some embodiments of the present disclosure, the above-mentioned crystal form G of the compound as shown in formula (IV) is prepared from the crystal form A of the compound as shown in formula (I) and methanesulfonic acid in 2-methyltetrahydrofuran. The ¹H NMR results show that the molar ratio of methanesulfonic acid to the compound (free base) as shown in formula (I) in the above-mentioned crystal form F of the compound as shown in formula (IV) is 0.8.

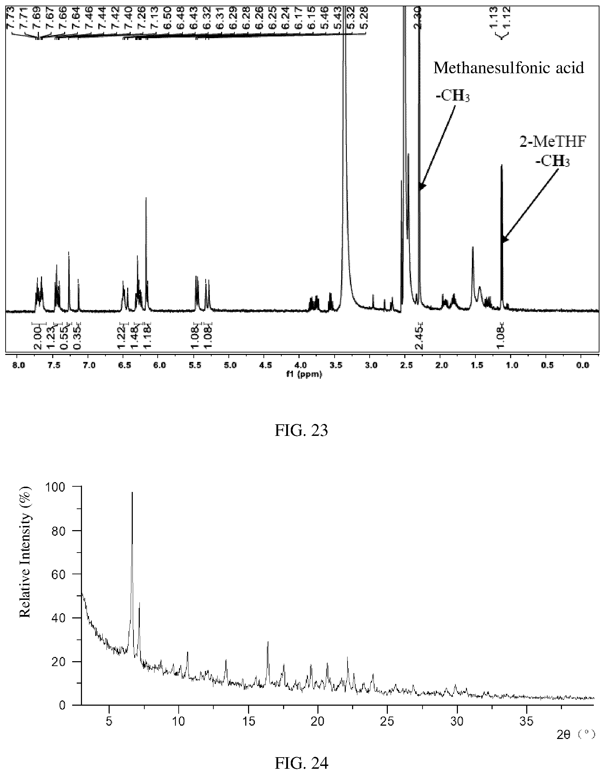

In some embodiments of the present disclosure, the ¹H NMR spectrum of the above-mentioned crystal form G of the compound as shown in formula (IV) is shown in FIG. 23.

The present disclosure provides a crystal form H of a compound as shown in formula (V) 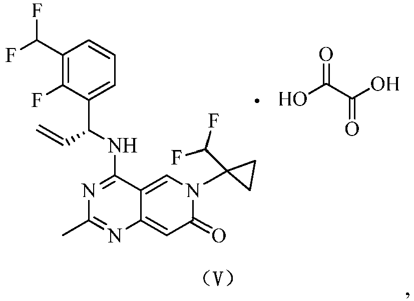 wherein an X-ray powder diffraction spectrum has diffraction peaks at the following 2θ angles: 6.64±0.20°, 7.14±0.20°, and 16.40±0.20°.

In some embodiments of the present disclosure, the above-mentioned crystal form H of the compound as shown in formula (V), wherein the X-ray powder diffraction spectrum has diffraction peaks at the following 2θ angles: 6.64±0.20°, 7.14±0.20°, 13.40±0.20°, 16.40±0.20°, 17.54±0.20°, 19.52±0.20°, 20.67±0.20°, and 22.15±0.20°.

In some embodiments of the present disclosure, the above-mentioned crystal form H of the compound as shown in formula (V), wherein the X-ray powder diffraction spectrum has diffraction peaks at the following 2θ angles: 6.64±0.20°, 7.14±0.20°, 10.62±0.20°, 11.94±0.20°, 13.40±0.20°, 15.55±0.20°, 16.40±0.20°, 17.54±0.20°, 18.49±0.20°, 19.52±0.20°, 20.67±0.20°, 22.15±0.20°, 22.59±0.20°, 23.27±0.20°, 23.97±0.20°, 25.56±0.20°, 26.84±0.20°, 29.18±0.20°, and 29.89±0.20°.

In some embodiments of the present disclosure, the above-mentioned crystal form H of the compound as shown in formula (V), wherein the X-ray powder diffraction spectrum has diffraction peaks at the following 2θ angles: 6.64°, 7.14°, 10.62°, 11.94°, 13.40°, 15.55°, 16.40°, 17.54°, 18.49°, 19.52°, 20.67°, 22.15°, 22.59°, 23.27°, 23.97°, 25.56°, 26.84°, 29.18°, and 29.89°.

In some embodiments of the present disclosure, the above-mentioned crystal form H of the compound as shown in formula (V), wherein the X-ray powder diffraction spectrum has diffraction peaks at the following 2θ angles: 6.64±0.20°, and/or 7.14±0.20°, and/or 10.62±0.20°, and/or 11.94±0.20°, and/or 13.40±0.20°, and/or 15.55±0.20°, and/or 16.40±0.20°, and/or 17.54±0.20°, and/or 18.49±0.20°, and/or 19.52±0.20°, and/or 20.67±0.20°, and/or 22.15±0.20°, and/or 22.59±0.20°, and/or 23.27±0.20°, and/or 23.97±0.20°, and/or 25.56±0.20°, and/or 26.84±0.20°, and/or 29.18±0.20°, and/or 29.89±0.20°.

In some embodiments of the present disclosure, the XRPD spectrum of the above-mentioned crystal form H of the compound as shown in formula (V) is shown in FIG. 24.

In some embodiments of the present disclosure, the XRPD spectrum analysis data of Cu Kα radiation of the above-mentioned crystal form H of the compound as shown in formula (V) is shown in Table 8:

**Table 8 XRPD diffraction peak data of the crystal form H of the compound as shown in formula (V)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 6.64 | 100.0 | 11 | 20.67 | 16.5 |
| 2 | 7.14 | 32.9 | 12 | 22.15 | 17.2 |
| 3 | 10.62 | 14.0 | 13 | 22.59 | 10.1 |
| 4 | 11.94 | 3.0 | 14 | 23.27 | 4.8 |
| 5 | 13.40 | 14.7 | 15 | 23.97 | 11.0 |
| 6 | 15.55 | 3.5 | 16 | 25.56 | 4.5 |
| 7 | 16.40 | 28.3 | 17 | 26.84 | 4.7 |
| 8 | 17.54 | 14.1 | 18 | 29.18 | 3.4 |
| 9 | 18.49 | 1.1 | 19 | 29.89 | 6.0 |
| 10 | 19.52 | 14.7 | | | |

In some embodiments of the present disclosure, a Differential Scanning Calorimetry (DSC) Curve of the above-mentioned crystal form H of the compound as shown in formula (V) has an endothermic peak at 214.6°C.

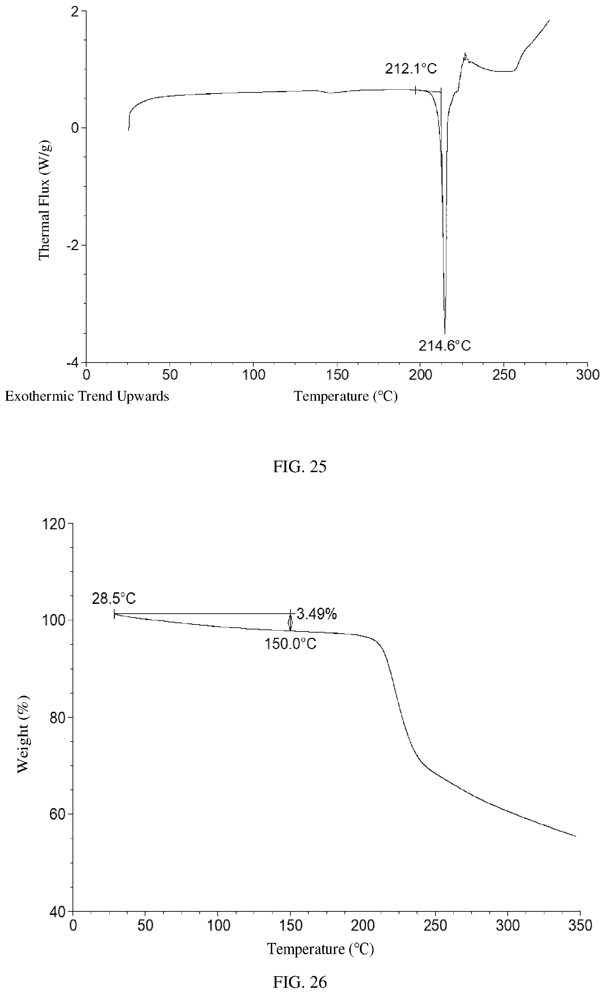

In some embodiments of the present disclosure, the DSC spectrum of the above-mentioned crystal form H of the compound as shown in formula (V) is shown in FIG. 25.

In some embodiments of the present disclosure, a Thermogravimetric Analysis (TGA) curve of the above-mentioned crystal form H of the compound as shown in formula (V) has a weight loss of 3.5% at 150.0±3°C.

In some embodiments of the present disclosure, the TGA spectrum of the above-mentioned crystal form H of the compound as shown in formula (V) is shown in FIG. 26.

In some embodiments of the present disclosure, the above-mentioned crystal form H of the compound as shown in formula (V) is prepared from the crystal form A of the compound as shown in formula (I) and oxalic acid in isopropyl alcohol. The UPLC/IC results show that the molar ratio of oxalic acid to the free base of the compound as shown in formula (I) is 1.0.

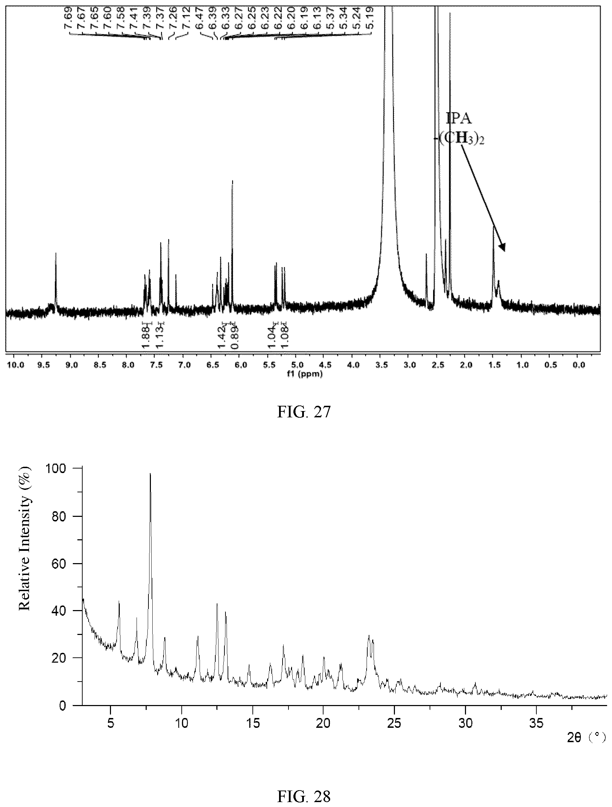

In some embodiments of the present disclosure, the ¹H NMR spectrum of the above-mentioned crystal form H of the compound as shown in formula (V) is shown in FIG. 27.

The present disclosure provides a crystal form I of a compound as shown in formula (V) 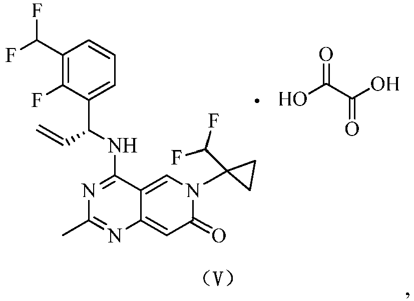 wherein an X-ray powder diffraction spectrum has diffraction peaks at the following 2θ angles: 7.81±0.20°, 12.50±0.20°, and 13.12±0.20°.

In some embodiments of the present disclosure, the above-mentioned crystal form I of the compound as shown in formula (V), wherein the X-ray powder diffraction spectrum has diffraction peaks at the following 2θ angles: 5.61±0.20°, 7.81±0.20°, 11.13±0.20°, 12.50±0.20°, 13.12±0.20°, 17.15±0.20°, 23.14±0.20°, and 23.49±0.20°.

In some embodiments of the present disclosure, the above-mentioned crystal form I of the compound as shown in formula (V), wherein the X-ray powder diffraction spectrum has diffraction peaks at the following 2θ angles: 5.61±0.20°, 6.85±0.20°, 7.81±0.20°, 8.82±0.20°, 11.13±0.20°, 12.50±0.20°, 13.12±0.20°, 14.75±0.20°, 16.26±0.20°, 17.15±0.20°, 17.76±0.20°, 18.55±0.20°, 19.36±0.20°, 20.04±0.20°, 21.24±0.20°, 22.48±0.20°, 23.14±0.20°, 23.49±0.20°, 24.50±0.20°, 25.35±0.20°, 28.24±0.20°, 30.67±0.20°, and 36.35±0.20°.

In some embodiments of the present disclosure, the above-mentioned crystal form I of the compound as shown in formula (V), wherein the X-ray powder diffraction spectrum has diffraction peaks at the following 2θ angles: 5.61°, 6.85°, 7.81°, 8.82°, 11.13°, 12.50°, 13.12°, 14.75°, 16.26°, 17.15°, 17.76°, 18.55°, 19.36°, 20.04°, 21.24°, 22.48°, 23.14°, 23.49°, 24.50°, 25.35°, 28.24°, 30.67°, and 36.35°.

In some embodiments of the present disclosure, the above-mentioned crystal form I of the compound as shown in formula (V), wherein the X-ray powder diffraction spectrum has diffraction peaks at the following 2θ angles: 5.61±0.20°, and/or 6.85±0.20°, and/or 7.81±0.20°, and/or 8.82±0.20°, and/or 11.13±0.20°, and/or 12.50±0.20°, and/or 13.12±0.20°, and/or 14.75±0.20°, and/or 16.26±0.20°, and/or 17.15±0.20°, and/or 17.76±0.20°, and/or 18.55±0.20°, and/or 19.36±0.20°, and/or 20.04±0.20°, and/or 21.24±0.20°, and/or 22.48±0.20°, and/or 23.14±0.20°, and/or 23.49±0.20°, and/or 24.50±0.20°, and/or 25.35±0.20°, and/or 28.24±0.20°, and/or 30.67±0.20°, and/or 36.35±0.20°.

In some embodiments of the present disclosure, the XRPD spectrum of the above-mentioned crystal form I of the compound as shown in formula (V) is shown in FIG. 28.

In some embodiments of the present disclosure, the XRPD spectrum analysis data of Cu Kα radiation of the above-mentioned crystal form I of the compound as shown in formula (V) is shown in Table 9:

**Table 9 XRPD diffraction peak data of the crystal form I of the compound as shown in formula (V)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 5.61 | 26.9 | 13 | 19.36 | 5.3 |
| 2 | 6.85 | 18.9 | 14 | 20.04 | 15.9 |
| 3 | 7.81 | 100.0 | 15 | 21.24 | 12.2 |
| 4 | 8.82 | 17.2 | 16 | 22.48 | 5.1 |
| 5 | 11.13 | 21.7 | 17 | 23.14 | 25.7 |
| 6 | 12.50 | 38.9 | 18 | 23.49 | 24.8 |
| 7 | 13.12 | 33.8 | 19 | 24.50 | 5.7 |
| 8 | 14.75 | 9.9 | 20 | 25.35 | 5.4 |
| 9 | 16.26 | 10.6 | 21 | 28.24 | 3.0 |
| 10 | 17.15 | 20.3 | 22 | 30.67 | 4.4 |
| 11 | 17.76 | 8.5 | 23 | 36.35 | 1.5 |
| 12 | 18.55 | 16.6 | | | |

In some embodiments of the present disclosure, a Differential Scanning Calorimetry (DSC) Curve of the above-mentioned crystal form I of the compound as shown in formula (V) has an endothermic peak at 112.9±3°C and 200.8±3°C, and an exothermic peak at 134.7±3°C.

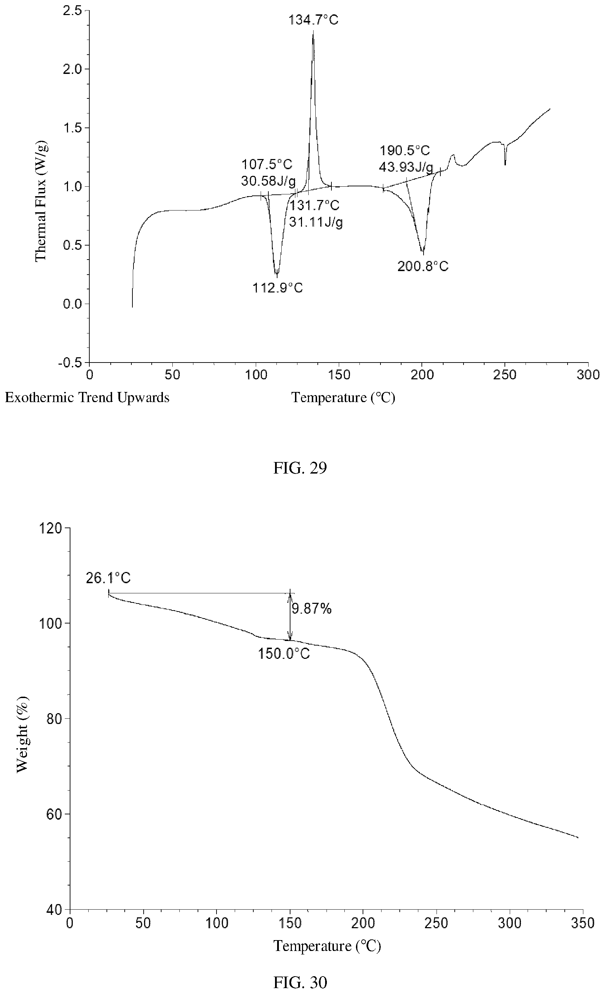

In some embodiments of the present disclosure, the DSC spectrum of the above-mentioned crystal form I of the compound as shown in formula (V) is shown in FIG. 29.

In some embodiments of the present disclosure, a Thermogravimetric Analysis (TGA) curve of the above-mentioned crystal form I of the compound as shown in formula (V) has a weight loss of 9.9% at 150.0±3°C.

In some embodiments of the present disclosure, the TGA spectrum of the above-mentioned crystal form I of the compound as shown in formula (V) is shown in FIG. 30.

In some embodiments of the present disclosure, the above-mentioned crystal form I of the compound as shown in formula (V) is prepared from the crystal form A of the compound as shown in formula (I) and oxalic acid in isopropyl acetate. The UPLC/IC results show that the molar ratio of oxalic acid to the free base of the compound as shown in formula (I) is 1.1.

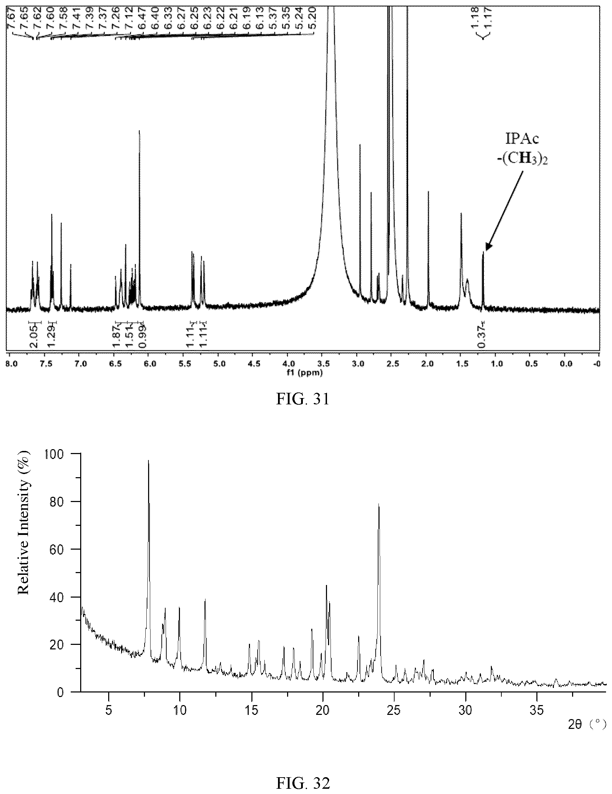

In some embodiments of the present disclosure, the ¹H NMR spectrum of the above-mentioned crystal form I of the compound as shown in formula (V) is shown in FIG. 31.

The present disclosure provides a crystal form J of a compound as shown in formula (V) 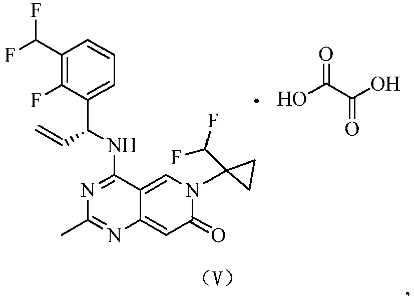 wherein an X-ray powder diffraction spectrum has diffraction peaks at the following 2θ angles: 7.80±0.20°, 20.27±0.20°, and 23.93±0.20°.

In some embodiments of the present disclosure, the above-mentioned crystal form J of the compound as shown in formula (V), wherein the X-ray powder diffraction spectrum has diffraction peaks at the following 2θ angles: 7.80±0.20°, 8.94±0.20°, 9.93±0.20°, 11.75±0.20°, 19.24±0.20°, 20.27±0.20°, 22.51±0.20°, and 23.93±0.20°.

In some embodiments of the present disclosure, the above-mentioned crystal form J of the compound as shown in formula (V), wherein the X-ray powder diffraction spectrum has diffraction peaks at the following 2θ angles: 7.80±0.20°, 8.94±0.20°, 9.93±0.20°, 11.75±0.20°, 12.81±0.20°, 14.84±0.20°, 15.50±0.20°, 15.91±0.20°, 17.25±0.20°, 17.96±0.20°, 18.41±0.20°, 19.24±0.20°, 19.87±0.20°, 20.27±0.20°, 20.47±0.20°, 21.72±0.20°, 22.51±0.20°, 23.05±0.20°, 23.38±0.20°, 23.93±0.20°, 25.13±0.20°, 25.75±0.20°, 26.50±0.20°, 27.05±0.20°, 27.64±0.20°, 30.02±0.20°, 31.03±0.20°, 31.83±0.20°, and 36.36±0.20°.

In some embodiments of the present disclosure, the above-mentioned crystal form J of the compound as shown in formula (V), wherein the X-ray powder diffraction spectrum has diffraction peaks at the following 2θ angles: 7.80°, 8.94°, 9.93°, 11.75°, 12.81°, 14.84°, 15.50°, 15.91°, 17.25°, 17.96°, 18.41°, 19.24°, 19.87°, 20.27°, 20.47°, 21.72°, 22.51°, 23.05°, 23.38°, 23.93°, 25.13°, 25.75°, 26.50°, 27.05°, 27.64°, 30.02°, 31.03°, 31.83°, and 36.36°.

In some embodiments of the present disclosure, the above-mentioned crystal form J of the compound as shown in formula (V), wherein the X-ray powder diffraction spectrum has diffraction peaks at the following 2θ angles: 7.80±0.20°, and/or 8.94±0.20°, and/or 9.93±0.20°, and/or 11.75±0.20°, and/or 12.81±0.20°, and/or 14.84±0.20°, and/or 15.50±0.20°, and/or 15.91±0.20°, and/or 17.25±0.20°, and/or 17.96±0.20°, and/or 18.41±0.20°, and/or 19.24±0.20°, and/or 19.87±0.20°, and/or 20.27±0.20°, and/or 20.47±0.20°, and/or 21.72±0.20°, and/or 22.51±0.20°, and/or 23.05±0.20°, and/or 23.38±0.20°, and/or 23.93±0.20°, and/or 25.13±0.20°, and/or 25.75±0.20°, and/or 26.50±0.20°, and/or 27.05±0.20°, and/or 27.64±0.20°, and/or 30.02±0.20°, and/or 31.03±0.20°, and/or 31.83±0.20°, and/or 36.36±0.20°.

In some embodiments of the present disclosure, the XRPD spectrum of the above-mentioned crystal form J of the compound as shown in formula (V) is shown in FIG. 32.

In some embodiments of the present disclosure, the XRPD spectrum analysis data of Cu Kα radiation of the above-mentioned crystal form J of the compound as shown in formula (V) is shown in Table 10:

**Table 10 XRPD diffraction peak data of the crystal form J of the compound as shown in formula (V)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 7.8047 | 100.00 | 16 | 21.72 | 3.2 |
| 2 | 8.9409 | 26.26 | 17 | 22.51 | 21.9 |
| 3 | 9.93 | 29.6 | 18 | 23.05 | 7.5 |
| 4 | 11.75 | 36.1 | 19 | 23.38 | 11.1 |
| 5 | 12.81 | 5.0 | 20 | 23.93 | 87.5 |
| 6 | 14.84 | 15.6 | 21 | 25.13 | 6.8 |
| 7 | 15.50 | 18.7 | 22 | 25.75 | 5.6 |
| 8 | 15.91 | 8.6 | 23 | 26.50 | 5.7 |
| 9 | 17.25 | 15.9 | 24 | 27.05 | 9.7 |
| 10 | 17.96 | 15.3 | 25 | 27.64 | 6.3 |
| 11 | 18.41 | 8.9 | 26 | 30.02 | 2.7 |
| 12 | 19.24 | 25.0 | 27 | 31.03 | 3.9 |
| 13 | 19.87 | 11.9 | 28 | 31.83 | 8.4 |
| 14 | 20.27 | 45.0 | 29 | 36.36 | 2.7 |
| 15 | 20.47 | 38.7 | | | |

In some embodiments of the present disclosure, a Differential Scanning Calorimetry (DSC) Curve of the above-mentioned crystal form J of the compound as shown in formula (V) has three endothermic peak at 90.3°C±3°C, 130.9°C±3°C, and 211.2°C±3°C, and one exothermic peak at 136.9°C.

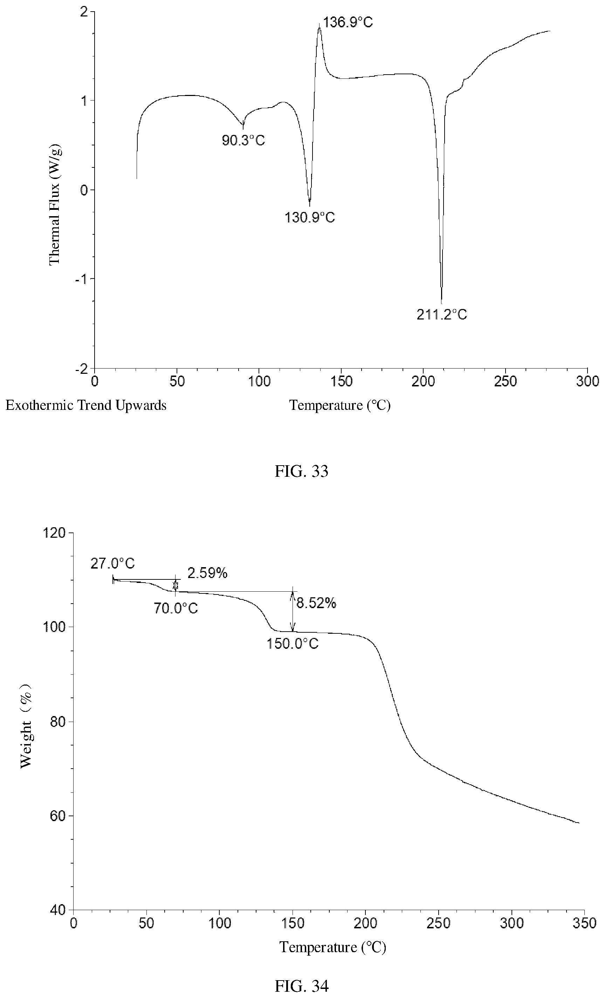

In some embodiments of the present disclosure, the DSC spectrum of the above-mentioned crystal form J of the compound as shown in formula (V) is shown in FIG. 33.

In some embodiments of the present disclosure, a Thermogravimetric Analysis (TGA) curve of the crystal form J of the compound as shown in formula (V) has a weight loss of 2.6% at 70.0±3°C, and after continued heating, the weight loss reaches 8.5% at 150.0±3°C.

In some embodiments of the present disclosure, the TGA spectrum of the above-mentioned crystal form J of the compound as shown in formula (V) is shown in FIG. 34.

In some embodiments of the present disclosure, the above-mentioned crystal form J of the compound as shown in formula (V) is prepared from the crystal form A of the compound as shown in formula (I) and oxalic acid in 2-methyltetrahydrofuran. The UPLC/IC results show that the molar ratio of oxalic acid to the free base of the compound as shown in formula (I) is 1.0.

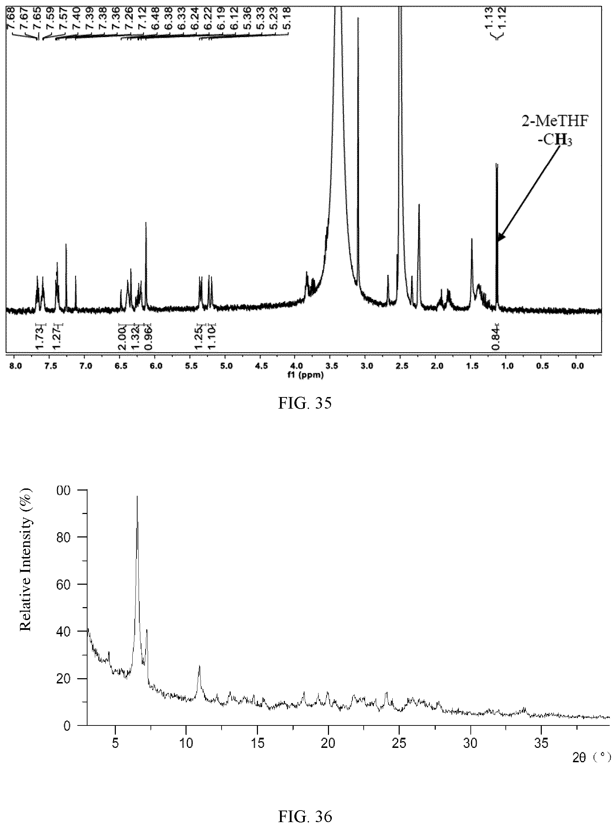

In some embodiments of the present disclosure, the ¹H NMR spectrum of the above-mentioned crystal form J of the compound as shown in formula (V) is shown in FIG. 35.

The present disclosure provides a crystal form K of a compound as shown in formula (VI) 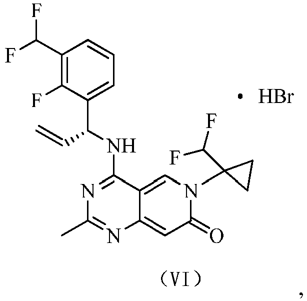 wherein an X-ray powder diffraction spectrum has diffraction peaks at the following 2θ angles: 6.54±0.20°, 7.24±0.20°, and 10.91±0.20°.

In some embodiments of the present disclosure, the above-mentioned crystal form K of the compound as shown in formula (VI), wherein the X-ray powder diffraction spectrum has diffraction peaks at the following 2θ angles: 6.54±0.20°, 7.24±0.20°, 10.91±0.20°, 18.26±0.20°, 19.30±0.20°, 19.95±0.20°, 21.78±0.20°, and 24.01±0.20°.

In some embodiments of the present disclosure, the above-mentioned crystal form K of the compound as shown in formula (VI), wherein the X-ray powder diffraction spectrum has diffraction peaks at the following 2θ angles: 4.50±0.20°, 6.54±0.20°, 7.24±0.20°, 10.91±0.20°, 13.07±0.20°, 15.48±0.20°, 18.26±0.20°, 19.30±0.20°, 19.95±0.20°, 21.78±0.20°, 23.23±0.20°, 24.01±0.20°, 25.81±0.20°, 27.74±0.20°, 31.50±0.20°, and 33.81±0.20°.

In some embodiments of the present disclosure, the above-mentioned crystal form K of the compound as shown in formula (VI), wherein the X-ray powder diffraction spectrum has diffraction peaks at the following 2θ angles: 4.50°, 6.54°, 7.24°, 10.91°, 13.07°, 15.48°, 18.26°, 19.30°, 19.95°, 21.78°, 23.23°, 24.01°, 25.81°, 27.74°, 31.50°, and 33.81°.

In some embodiments of the present disclosure, the above-mentioned crystal form K of the compound as shown in formula (VI), wherein the X-ray powder diffraction spectrum has diffraction peaks at the following 2θ angles: 6.54±0.20°, and/or 4.50±0.20°, and/or 7.24±0.20°, and/or 10.91±0.20°, and/or 13.07±0.20°, and/or 15.48±0.20°, and/or 18.26±0.20°, and/or 19.30±0.20°, and/or 19.95±0.20°, and/or 21.78±0.20°, and/or 23.23±0.20°, and/or 24.01±0.20°, and/or 25.81±0.20°, and/or 27.74±0.20°, and/or 31.50±0.20°, and/or 33.81±0.20°.

In some embodiments of the present disclosure, the XRPD spectrum analysis data of Cu Kα radiation of the above-mentioned crystal form K of the compound as shown in formula (VI) is shown in Table 11:

**Table 11 XRPD diffraction peak data of the crystal form K of the compound as shown in formula (VI)**

| **Serial Number** | **2θ angle (±0.20°)** | **Relative Intensity(%)** | **Serial Number** | **2θ angle (±0.20°)** | **Relative Intensity(%)** |
|---|---|---|---|---|---|
| 1 | 4.50 | 4.7 | 9 | 19.95 | 8.8 |
| 2 | 6.54 | 100.0 | 10 | 21.78 | 7.5 |
| 3 | 7.24 | 29.2 | 11 | 23.23 | 3.8 |
| 4 | 10.91 | 18.9 | 12 | 24.01 | 8.4 |
| 5 | 13.07 | 5.1 | 13 | 25.81 | 4.9 |
| 6 | 15.48 | 3.2 | 14 | 27.74 | 5.0 |
| 7 | 18.26 | 6.3 | 15 | 31.50 | 1.3 |
| 8 | 19.30 | 7.2 | 16 | 33.81 | 2.9 |

In some embodiments of the present disclosure, the XRPD spectrum of the above-mentioned crystal form K of the compound as shown in formula (VI) is shown in FIG. 36.

In some embodiments of the present disclosure, a Differential Scanning Calorimetry (DSC) Curve of the above-mentioned crystal form K of the compound as shown in formula (VI) has one endothermic peak at 94.7°C±3°C.

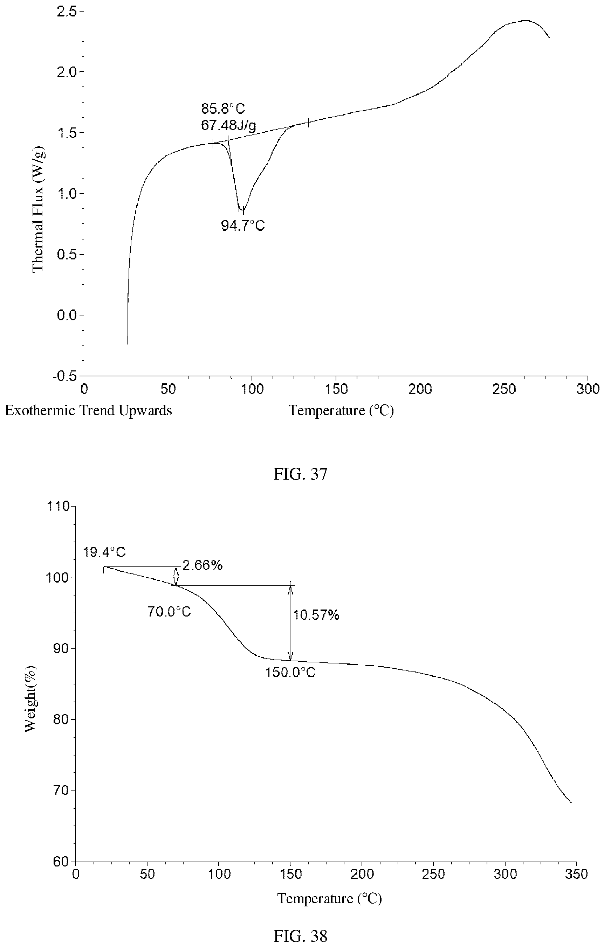

In some embodiments of the present disclosure, the DSC spectrum of the above-mentioned crystal form K of the compound as shown in formula (VI) is shown in FIG. 37.

In some embodiments of the present disclosure, a Thermogravimetric Analysis (TGA) curve of the crystal form K of the compound as shown in formula (VI) has a weight loss of 2.7% at 70.0±3°C, and after continued heating, the weight loss reaches 10.6% at 150.0±3°C.

In some embodiments of the present disclosure, the TGA spectrum of the above-mentioned crystal form K of the compound as shown in formula (VI) is shown in FIG. 38.

In some embodiments of the present disclosure, the above-mentioned crystal form K of the compound as shown in formula (VI) is prepared from the crystal form A of the compound as shown in formula (I) and hydrogen bromide in isopropyl alcohol. The UPLC/IC results show that the molar ratio of hydrogen bromide to the compound (free base) as shown in formula (I) is 1.0.

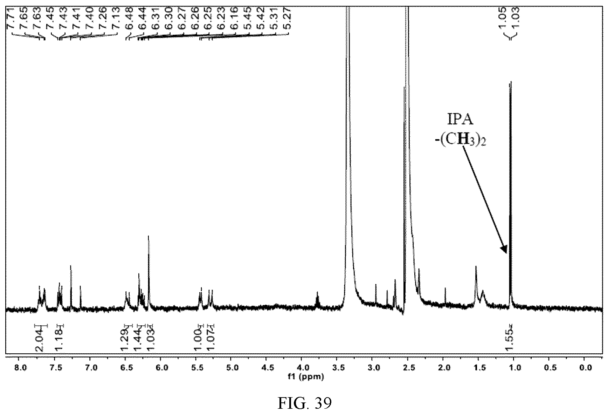

In some embodiments of the present disclosure, the ¹H NMR spectrum of the above-mentioned crystal form K of the compound as shown in formula (VI) is shown in FIG. 39.

The present disclosure also provides application of the above-mentioned crystal form A of the compound as shown in formula (I), and/or the crystal form B of the compound as shown in formula (I), and/or the crystal form C of the compound as shown in formula (I), and/or the crystal form D of the compound as shown in formula (II), and/or the crystal form E of the compound as shown in formula (III), and/or the crystal form F of the compound as shown in formula (IV), and/or the crystal form G of the compound as shown in formula (IV), and/or the crystal form H of the compound as shown in formula (V), and/or the crystal form I of the compound as shown in formula (V), and/or the crystal form J of the compound as shown in formula (V), and/or the crystal form K of the compound as shown in formula (VI) in preparation of the treatment of lung cancer, pancreatic cancer or rectal cancer.

### Definition and Description

Unless otherwise stated, the following terms and phrases used herein are intended to have the following meanings. A particular phrase or term should not be considered uncertain or unclear in the absence of a specific definition, but should be understood in its ordinary meaning. When a trade name appears herein, it is intended to refer to its corresponding commodity or its active ingredient.

The intermediate compounds of the present disclosure can be prepared by a variety of synthetic methods well known to those skilled in the art, including the specific embodiments listed below, embodiments formed by combining them with other chemical synthesis methods, and equivalent replacements well known to those skilled in the art, and preferred embodiments include, but are not limited to, embodiments of the present disclosure.

The chemical reactions of the specific embodiments of the present disclosure are completed in a suitable solvent, and the solvent must be suitable for the chemical changes of the present disclosure and the required reagents and materials. In order to obtain the compounds of the present disclosure, those skilled in the art sometimes need to modify or select the synthesis steps or reaction procedures based on the existing embodiments.

The present disclosure will be described in detail through embodiments below. These embodiments do not mean any limitation to the present disclosure.

All solvents used in the present disclosure are commercially available and used without further purification.

The following abbreviations are used in the present disclosure: DMSO represents dimethyl sulfoxide; DMF represents N,N-dimethylformamide; DCM represents dichloromethane; dioxane or 1,4-dioxane represents 1,4-dioxane; Ts represents 4-methylbenzenesulfonyl; TsCI represents 4-methylbenzenesulfonyl chloride; Boc represents tert-butoxycarbonyl, which is a protective group for amino groups; LC-MS represents liquid chromatography-mass spectrometry; HPLC represents liquid chromatography; UPLC represents ultra-performance liquid chromatography; IC represents ion chromatography; mg represents milligram; µg represents microgram; ng represents nanogram; µL represents microliter; mL represents milliliter; mm represents millimeter; µm represents micrometer; mM represents millimole/liter, µM represents micromole/liter; nM represents nanomole/liter; h represents hour; and min represents minutes.

Compounds are named according to conventional naming principles in the field or using ChemDraw^{®} software, and commercially available compounds use supplier catalog names.

### BRIEF DESCRIPTION OF THE DRAWINGS

FIG. 1 is a XRPD diagram of crystal form A of a compound as shown in formula (I).
FIG. 2 is a DSC diagram of crystal form A of a compound as shown in formula (I).
FIG. 3 is a TGA diagram of crystal form A of a compound as shown in formula (I).
FIG. 4 is a XRPD diagram of crystal form B of a compound as shown in formula (I).
FIG. 5 is a DSC diagram of crystal form B of a compound as shown in formula (I).
FIG. 6 is a TGA diagram of crystal form B of a compound as shown in formula (I).
FIG. 7 is a XRPD diagram of crystal form C of a compound as shown in formula (I).
FIG. 8 is a XRPD diagram of crystal form D of a compound as shown in formula (II).
FIG. 9 is a DSC diagram of crystal form D of a compound as shown in formula (II).
FIG. 10 is a TGA diagram of crystal form D of a compound as shown in formula (II).
FIG. 11 is a ¹H NMR diagram of crystal form D of a compound as shown in formula (II).
FIG. 12 is a XRPD diagram of crystal form E of a compound as shown in formula (III).
FIG. 13 is a DSC diagram of crystal form E of a compound as shown in formula (III).
FIG. 14 is a TGA diagram of crystal form E of a compound as shown in formula (III).
FIG. 15 is a ¹H NMR diagram of crystal form E of a compound as shown in formula (III).
FIG. 16 is a XRPD diagram of crystal form F of a compound as shown in formula (IV).
FIG. 17 is a DSC diagram of crystal form F of a compound as shown in formula (IV).
FIG. 18 is a TGA diagram of crystal form F of a compound as shown in formula (IV).
FIG. 19 is a ¹H NMR diagram of crystal form F of a compound as shown in formula (IV).
FIG. 20 is a XRPD diagram of crystal form G of a compound as shown in formula (IV).
FIG. 21 is a DSC diagram of crystal form G of a compound as shown in formula (IV).
FIG. 22 is a TGA diagram of crystal form G of a compound as shown in formula (IV).
FIG. 23 is a ¹H NMR diagram of crystal form G of a compound as shown in formula (IV).
FIG. 24 is a XRPD diagram of crystal form H of a compound as shown in formula (V).
FIG. 25 is a DSC diagram of crystal form H of a compound as shown in formula (V).
FIG. 26 is a TGA diagram of crystal form H of a compound as shown in formula (V).
FIG. 27 is a ¹H NMR diagram of crystal form H of a compound as shown in formula (V).
FIG. 28 is a XRPD diagram of crystal form I of a compound as shown in formula (V).
FIG. 29 is a DSC diagram of crystal form I of a compound as shown in formula (V).
FIG. 30 is a TGA diagram of crystal form I of a compound as shown in formula (V).
FIG. 31 is a ¹H NMR diagram of crystal form I of a compound as shown in formula (V).
FIG. 32 is a XRPD diagram of crystal form J of a compound as shown in formula (V).
FIG. 33 is a DSC diagram of crystal form J of a compound as shown in formula (V).
FIG. 34 is a TGA diagram of crystal form J of a compound as shown in formula (V).
FIG. 35 is a ¹H NMR diagram of crystal form J of a compound as shown in formula (V).
FIG. 36 is a XRPD diagram of crystal form K of a compound as shown in formula (VI).
FIG. 37 is a DSC diagram of crystal form K of a compound as shown in formula (VI).
FIG. 38 is a TGA diagram of crystal form K of a compound as shown in formula (VI).
FIG. 39 is a ¹H NMR diagram of crystal form K of a compound as shown in formula (VI).

### DETAILED DESCRIPTION OF THE EMBODIMENTS

The present disclosure is described in detail below through embodiments, which do not mean any adverse limitations to the present disclosure. The compounds of the present disclosure can be prepared by a variety of synthetic methods well known to those skilled in the art, including the specific embodiments listed below, embodiments formed by combining them with other chemical synthesis methods, and equivalent replacements well known to those skilled in the art, and preferred embodiments include, but are not limited to, embodiments of the present disclosure. It will be apparent to those skilled in the art that various changes and modifications can be made in the specific embodiments of the present disclosure without departing from the spirit and scope of the present disclosure.

### Embodiment 1 Preparation of compounds as shown in formula (I)

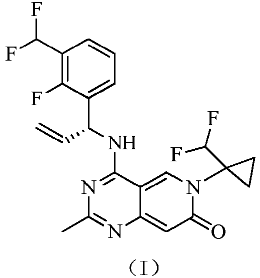

### Preparation of intermediates 1-10

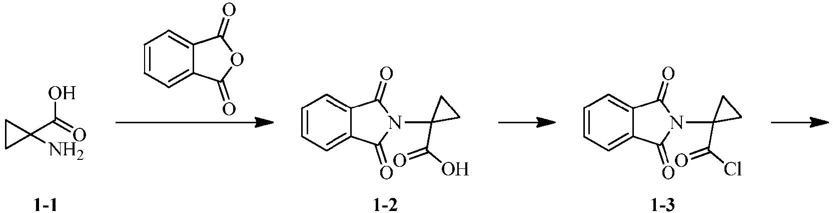

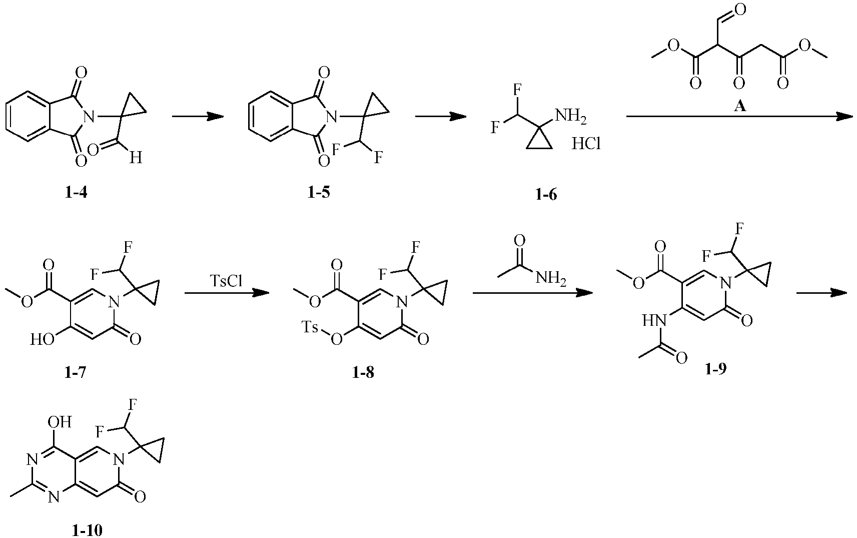

Step A: **1-1** (22.3 g, 220.57 mmol) and phthalic anhydride (32.67 g, 220.57 mmol) were added to glacial acetic acid (200 mL). Subsequently, the reaction mixture was stirred at 110°C for 3 h. After concentration under reduced pressure to remove acetic acid, water (200 mL) was added, and the mixture was stirred at 15°C for 1 h. The precipitate was then filtered, and the filter cake was collected and dried under reduced pressure to obtain **1-2.** ¹H NMR (400 MHz, DMSO-*d₆*) *δ*=13.60-12.40 (m, 1H), 7.95-7.82 (m, 4H), 1.66-1.59 (m, 2H), and 1.47-1.40 (m, 2H).

Step B: **1-2** (10 g, 43.25 mmol) and N,N-dimethylformamide (158.07 mg, 2.16 mmol) were added to toluene (30 mL) at room temperature, followed by the slow addition of thionyl chloride (5.40 g, 45.41 mmol). After the addition was complete, the reaction mixture was stirred at 110°C for 3 h. The solvent was removed by concentration under reduced pressure to obtain **1-3,** which was directly used in the next reaction.

Step C: **1-3** (20.5 g, 82.12 mmol) and 2,6-lutidine (10.56 g, 98.54 mmol) were added to tetrahydrofuran (200 mL), followed by the addition of dry palladium on carbon (10%, 1.02 g). The reaction mixture was stirred under hydrogen at 45 psi and 30°C for 20 h. Subsequently, without further addition of palladium (10%, 1.02 g), stirring was continued under hydrogen at 45 psi and 30°C for an additional 24 h. After filtration, the filtrate was concentrated under reduced pressure. The residue was separated and purified by silica gel column chromatography (eluent: V/V petroleum ether/ethyl acetate=10/1-3/1 + 20% dichloromethane) to obtain **1-4.** ¹H NMR (400 MHz, CDCl3) *δ*=8.88 (s, 1H), 7.89-7.82 (m, 2H), 7.77-7.71 (m, 2H), 1.86-1.79 (m, 2H), 1.72-1.65 (m, 2H).

Step D: **1-4** (7.54 g, 35.04 mmol) was dissolved in dichloromethane (45 mL), followed by the slow addition of dichloromethane (20 mL) solution of bis(2-methoxyethyl)aminosulfur trifluoride (18.60 g, 84.09 mmol). After the addition was complete, the reaction mixture was stirred at 20°C for 48 h. The reaction was quenched with saturated aqueous sodium carbonate solution (100 mL) and stirred for 1 h at 10°C. Water (100 mL) was then added, and the mixture was extracted with dichloromethane (100 mL/time, extraction 3 times) The combined organic phases were dried over anhydrous sodium sulfate. After filtration, the filtrate was concentrated under reduced pressure. The residue was separated and purified by silica gel column chromatography (eluent: V/V petroleum ether/ethyl acetate=15/1-10/1) to obtain 1-5. ¹H NMR (400 MHz, CDCl₃) *δ*=7.88 (dd, *J*=3.2, 5.6 Hz, 2H), 7.76 (dd, *J*=3.2, 5.6 Hz, 2H), 6.07-5.69 (m, 1H), 1.47-1.41 (m, 2H), 1.27-1.20 (m, 2H).

Step E: **1-5** (7.1 g, 29.93 mmol) was added to 2-(2-aminoethylamino)ethanol (21.30 mL). The reaction mixture was stirred at 80°C for 2 h. Methanol (150 mL) was then added, and the mixture was distilled at 90°C under atmospheric pressure, with the distillate being cooled with a dry ice bath. A solution of hydrogen chloride in methanol (4 mol/L, 15 mL) was added to the distillate. After concentration under reduced pressure, **1-6** was obtained. ¹H NMR (400 MHz, CD₃OD) *δ*=6.04-5.71 (m, 1H), 1.24 (s, 4H).

Step F: **1-6** (4 g, 27.86 mmol) and compound **A** (6.76 g, 33.43 mmol) were added to methanol (40 mL), followed by sodium methoxide in methanol (25%, 5.42 g). After the reaction mixture was stirred at 10°C for 17 h, a methanol solution of sodium methoxide (25%, 7.22 g) was added, and stirring was continued for 2 h. Methanol was removed by concentration under reduced pressure, and the residue was acidified to pH=6-7 with concentrated hydrochloric acid (12 mol/L). After dilution with water (100 mL), the mixture was extracted with ethyl acetate (100 mL/time, extraction 3 times). The combined organic phases were dried over anhydrous sodium sulfate. After filtration, the filtrate was concentrated under reduced pressure. The residue was stirred in tert-butyl methyl ether (100 mL) for 1 h, filtered, and the filter cake was collected to obtain **1-7.** ¹H NMR (400 MHz, DMSO-*d₆*) *δ*=10.93 (s, 1H), 8.09 (s, 1H), 6.32-5.98 (m, 1H), 5.68 (s, 1H), 3.80 (s, 3H), 1.41-1.23 (m, 4H).

Step G: **1-7** (5.17 g, 19.95 mmol) and 4-methylbenzenesulfonyl chloride (4.58 g, 24.02 mmol) were added to acetonitrile (50 mL), followed by the addition of triethylamine (3.03 g, 29.92 mmol). The reaction mixture was stirred at 20°C for 2 h. After dilution with water (50 mL), the mixture was filtered The filter cake was washed with water (30 mL/time, washed 3 times), and then dried under vacuum to obtain **1-8.** ¹H NMR (400 MHz, DMSO-*d₆*) *δ*=8.15 (s, 1H), 7.84 (d, *J*=8.4 Hz, 2H), 7.52 (d, *J*=8.4 Hz, 2H), 6.31-5.98 (m, 2H), 3.67 (s, 3H), 2.44 (s, 3H), 1.43-1.30 (m, 4H).

Step H: **1-8** (7.41 g, 17.92 mmol), acetamide (1.06 g, 17.92 mmol), potassium phosphate (4.19 g, 19.72 mmol), di-chlorobis[(1,2,3-)-1-phenyl-2-propenyl]dipalladium(II) (187.90 mg, 358.50 µmol) and Xantphos (414.87 mg, 717.00 µmol) were added in one portion to 1,4-dioxane (35 mL). The reaction mixture was protected by nitrogen and stirred at 115°C for 12 h. After natural cooling, water (100 mL) was added to dilute the reaction solution, which was then extracted with dichloromethane (100 mL/time, 3 times). The combined organic phases were dried over anhydrous sodium sulfate. After filtration, the filtrate was concentrated under reduced pressure. The residue was separated and purified by silica gel column chromatography (eluent: V/V dichloromethane/ethyl acetate=10/1-5/1) to obtain **1-9.** ¹H NMR (400 MHz, CDCl₃) *δ*=10.79 (br s, 1H), 8.23 (s, 1H), 7.69 (s, 1H), 6.40-6.03 (m, 1H), 3.89 (s, 3H), 2.21 (s, 3H), 1.57-1.50 (m, 2H), 1.19-1.11 (m, 2H).

Step I: **1-9** (4.22 g, 14.05 mmol) was added to methanol solution of ammonia (7 mol/L, 34 mL). The reaction mixture was stirred at 15°C for 3 days. After concentration under reduced pressure, an aqueous sodium hydroxide solution (1 mol/L, 30 mL) was added to the residue, followed by stirring at 50°C for 30 min. After cooling naturally, the solution was washed with tert-butyl methyl ether (70 mL/time, washed 3 times). The aqueous phase was acidified with concentrated hydrochloric acid (12 mol/L) to a pH of approximately 4, resulting in the precipitation of a white solid. The solid was filtered, the filter cake was collected, and it was dried under reduced pressure to obtain **1-10.** ¹H NMR (400 MHz, DMSO-*d₆*) *δ*=11.85 (br s, 1H), 8.36 (s, 1H), 6.38-6.05 (m, 2H), 2.24 (s, 3H), 1.50-1.29 (m, 4H).

### Preparation of intermediates 1-16

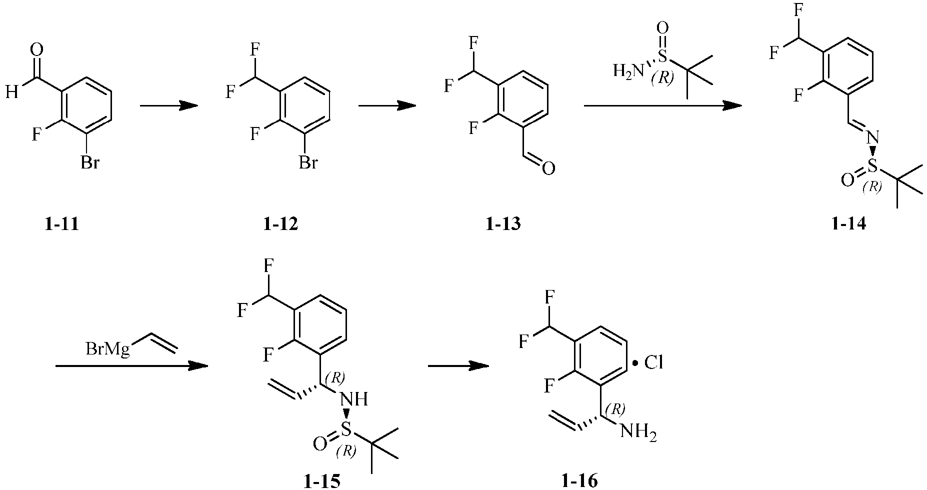

Step A: at 0°C, diethylamino sulfur trifluoride (15.88 g, 98.52 mmol) was added dropwise to a solution of **1-11** (10.00 g, 49.26 mmol) in dichloromethane (100 mL). The reaction mixture was stirred at 0°C for 1 h, then a saturated aqueous sodium bicarbonate solution was added slowly until the pH reached 8. The mixture was extracted with dichloromethane (50 mL/time, three times), and the combined organic phases were dried over anhydrous sodium sulfate. After filtration, the solution was concentrated under reduced pressure to obtain crude **1-12,** which was directly used in the next step of the reaction.

Step B: under nitrogen protection at -78°C, n-butyllithium (2.5 mol per liter, 13.33 mL, 33.33 mmol) was added dropwise to a solution of **1-12** (5.00 g, 22.22 mmol) in tetrahydrofuran (50 mL). After the addition was completed, the reaction mixture was stirred at -78°C for 1 h, then N,N-dimethylformamide (3.25 g, 44.44 mmol) was added, and stirring was continued at -78°C for 1 h. The reaction mixture was then poured into dilute hydrochloric acid (1 mol/L, 100 mL), and extracted with ethyl acetate (30 mL/time, three times). The combined organic phases were dried over anhydrous sodium sulfate, filtered, and concentrated under reduced pressure to obtain crude product **1-13,** which was directly used in the next reaction.

Step C: cesium carbonate (5.99 g, 18.38 mmol) and (R)-tert-butanesulfinamide (2.23 grams, 18.38 mmol) were added to a solution of **1-13** (3.20 g, 18.38 mmol) in tetrahydrofuran (60 mL). The reaction mixture was stirred at 0°C for 1 h, then diluted with water (200 mL), and extracted with ethyl acetate (100 mL/time, twice). The combined organic phases were dried over anhydrous sodium sulfate, filtered, and concentrated under reduced pressure. The residue was subjected to preparative HPLC (column: Phenomenex luna C18, 250×50 mm×10 µm; mobile phase: phase A: 0.225% formic acid aqueous solution; phase B: acetonitrile, 40-70%; 18 min) to obtain **1-14.** ¹H NMR (400 MHz, DMSO-*d₆*) *δ*=8.72 (s, 1H), 8.19 (t, *J*=7.0 Hz, 1H), 7.89 (t, *J*=7.0 Hz, 1H), 7.52 (t, *J*=7.8 Hz, 1H), 7.48-7.14 (m, 1H), 1.21 (s, 9H).

Step D: under nitrogen protection at 20°C, vinylmagnesium bromide solution (1 mol/L, 3.61 mmol) was added dropwise to dimethylzinc solution (1 mol/L, 4.15 mmol). After the addition was complete, the reaction mixture was stirred at 20°C for 4 h and then cooled to -78°C. Subsequently, a solution of **1-14** (0.50 g, 1.80 mmol) in tetrahydrofuran (10 mL) was slowly added dropwise. After completion, continue stirring at -78°C for 0.5 h. The reaction mixture was quenched with saturated aqueous ammonium chloride solution (200 mL), and then extracted with ethyl acetate (300 mL/time, twice). The combined organic phases were washed once with saturated sodium chloride (300 mL/time) and dried over anhydrous sodium sulfate. After filtration, the filtrate was concentrated under reduced pressure. The residue was purified by column silica gel chromatography (eluent: V/V, petroleum ether/ethyl acetate=5/1-3/1) to obtain **1-15.** ¹H NMR (400 MHz, DMSO-*d₆*) *δ*=7.70 (t, *J*=7.0 Hz, 1H), 7.56 (t, *J*=7.0 Hz, 1H), 7.42-7.07 (m, 2H), 6.04-5.95 (m, 1H), 5.35-5.08 (m, 3H), 1.13 (s, 9H).

Step E: 1,4-dioxane solution of hydrogen chloride (4 mol/L, 0.98 mL) was added to a solution of **1-15** (0.40 g, 1.31 mmol) in tetrahydrofuran (5 mL). After the mixture was stirred at 15-20°C for 2 h, petroleum ether (40 mL) was added and stirring continued for 30 min. After filtration, the filter cake was collected. After drying under reduced pressure, **1-16** was obtained.

### Preparation of compounds as shown in formula (I)

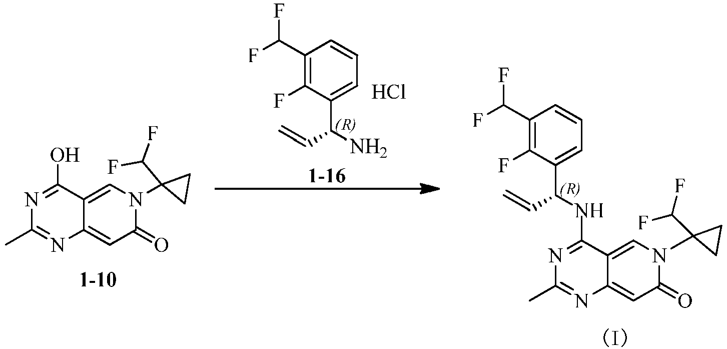

**1-10** (700 mg, 2.62 mmol), phosphonitrilic chloride trimer (867.31 mg, 2.50 mmol), and potassium phosphate (1.32 g, 6.24 mmol) were added to acetonitrile (20 mL), and the mixture was stirred at 155°C for 1 h. Subsequently, **1-16** (593 mg, 2.50 mmol) was added, and stirring was continued for another 1 h. After removing the solvent by concentration under reduced pressure, the residue was separated and purified by preparative HPLC (column: Waters Xbridge BEH C18 250×50 mm×10 µm; mobile phase: Phase A: aqueous ammonia; Phase B: acetonitrile, 35%-65%; 20 min) to obtain the compound represented by formula (I). ¹H NMR (400 MHz, CD₃OD) *δ* =9.26 (s, 1H), 7.63 (t, *J*=7.2 Hz, 1H), 7.55 (t, *J*=7.0 Hz, 1H), 7.30 (t, *J*=7.8 Hz, 1H), 7.17-6.85 (m, 1H), 6.43 (d, *J*=6.0 Hz, 1H), 6.35 (s, 1H), 6.25 (ddd, *J*=6.0, 10.4, 17.0 Hz, 1H), 5.35 (d, *J*=10.4 Hz, 1H), 5.26 (d, *J*=17.0 Hz, 1H), 2.33 (s, 3H), 1.59 (s, 3H), 1.27-1.07 (m, 4H). LC-MS (ESI) m/z: 451.2 [M+H]⁺.

### Embodiment 2 Preparation of crystal form A of the compound as shown in formula (I)

The compound as shown in formula (I) (450 mg, 1 mmol) was added to tert-butyl acetate (2.5 mL) and stirred at 60°C for 1 h. After natural cooling, the mixture was filtered under reduced pressure to obtain crystal form A of the compound as shown in formula (I).

### Embodiment 3 Preparation of crystal form B of the compound as shown in formula (I)

Approximately 20 mg of the compound as shown in formula (I) was weighed and added to a 20 mL vial. The solid was completely dissolved using 0.2 mL of acetonitrile. While stirring the clear solution at 1000 rpm, water was added dropwise until solid precipitation occurred. After filtration, the solid was dried under vacuum to obtain crystal form B of the compound as shown in formula (I).

### Embodiment 4 Preparation of crystal form C of the compound as shown in formula (I)

The crystal form B of the compound as shown in formula (I) was heated to 180°C under a nitrogen atmosphere to obtain the crystal form C of the compound as shown in formula (I).

### Embodiment 5 Preparation of crystal form D of the compound as shown in formula (II)

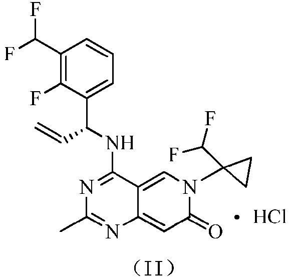

The crystal form A of the compound as shown in formula (I) and an equimolar amount of hydrochloric acid were stirred in isopropyl alcohol at room temperature for 2 days, filtered and dried under reduced pressure to obtain the crystal form D of the compound as shown in formula (II).

### Embodiment 6 Preparation of crystal form E of the compound as shown in formula (III)

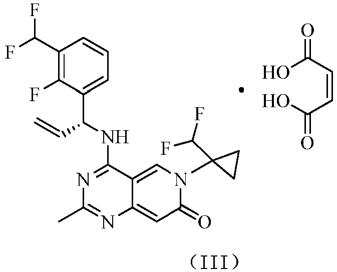

The crystal form A of the compound as shown in formula (I) and an equimolar amount of maleic acid were stirred in isopropyl alcohol at room temperature for 2 days, filtered and dried under reduced pressure to obtain the crystal form E of the compound as shown in formula (III).

### Embodiment 7 Preparation of crystal form F of the compound as shown in (IV)

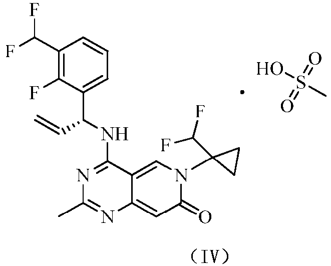

The crystal form A of the compound as shown in formula (I) and an equimolar amount of methanesulfonic acid were stirred in isopropyl acetate at room temperature for 2 days, filtered and dried under reduced pressure to obtain the crystal form F of the compound as shown in formula (IV).

### Embodiment 8 Preparation of crystal form G of the compound as shown in (IV)

The crystal form A of the compound as shown in formula (I) and an equimolar amount of methanesulfonic acid were stirred in 2-methyltetrahydrofuran at room temperature for 2 days, filtered and dried under reduced pressure to obtain the crystal form G of the compound as shown in formula (IV).

### Embodiment 9 Preparation of crystal form H of the compound as shown in formula (V)

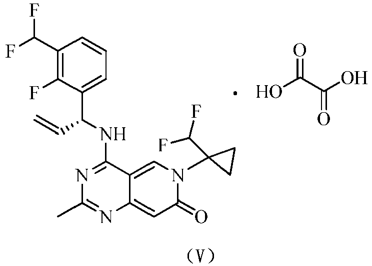

The crystal form A of the compound as shown in formula (I) and an equimolar amount of oxalic acid were stirred in isopropyl alcohol at room temperature for 2 days, filtered and dried under reduced pressure to obtain the crystal form H of the compound as shown in formula (V).

### Embodiment 10 Preparation of crystal form I of the compound as shown in formula (V)

The crystal form A of the compound as shown in formula (I) and an equimolar amount of oxalic acid were stirred in isopropyl acetate at room temperature for 2 days, filtered and dried under reduced pressure to obtain the crystal form I of the compound as shown in formula (V).

### Embodiment 11 Preparation of crystal form J of the compound as shown in formula (V)

The crystal form A of the compound as shown in formula (I) and an equimolar amount of oxalic acid were stirred in 2-methyltetrahydrofuran at room temperature for 2 days, filtered and dried under reduced pressure to obtain the crystal form J of the compound as shown in formula (V).

### Embodiment 12 Preparation of crystal form K of the compound as shown in formula (VI)

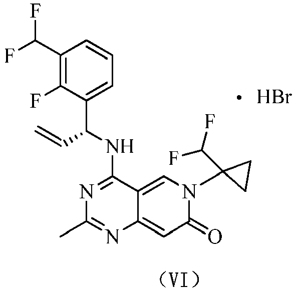

The crystal form A of the compound as shown in formula (I) and an equimolar amount of hydrogen bromide were stirred in isopropyl alcohol at room temperature for 2 days, filtered and dried under reduced pressure to obtain the crystal form K of the compound as shown in formula (VI).

### Experimental Embodiment 1. KRAS (G12C) and SOS1 binding experiment

### 1. Experimental materials:

The KRAS (G12C) protein was expressed and purified by Wuhan AtaGenix Biotechnology Co., Ltd. The SOS1 exchange domain (564-1049) protein (Human recombinant) was purchased from Cytoskeleton, Mab Anti 6HIS-XL665 and Mab Anti GST-Eu cryptate were purchased from Cisbio. The multi-functional microplate reader Nivo5 was purchased from PerkinElmer.

### 2. Preparation of 1× buffer:

The buffer solution in this experiment was prepared and used immediately.

Preparation method: 4-hydroxyethylpiperazine ethyl sulfonate (Hepes): 5 mM; sodium chloride (NaCl): 150 mM; ethylenediaminetetraacetic acid (EDTA): 10 mM; CO-630 (Igepal): 0.0025%; potassium fluoride (KF): 100 mM; dithiothreitol (DTT): 1 mM; and bovine serum albumin (BSA): 0.05%.

### 3. Experimental methods:

1) The test compound was diluted five-fold using DMSO with a multi-channel pipette until the 8th concentration was achieved, starting from 1 mM and ending at 0.064 µM.
2) The various gradients of the test compound were diluted with 1× buffer to form working solutions containing 2% DMSO. 5 µL of each working solution was then dispensed into the corresponding wells, with a concentration gradient ranging from 20 µM to 0.00128 nM. Duplicate well experiment was set up for each concentration. Following this, the plate was centrifuged at 1000 rpm for 1 min.
3) A mixed working solution containing KRAS (G12C) at a concentration of 200 nM and Mab Anti GST-Eu cryptate at a concentration of 1 ng/µL was prepared using 1× buffer. This mixed working solution was then incubated at 25°C for 5 min before 2.5 µL of it was dispensed into the corresponding wells.
4) A mixed working solution containing SOS1 at a concentration of 80 nM and Mab Anti 6HIS-XL665 at a concentration of 8 g/µL was prepared using 1× buffer. 2.5 µL of this mixed solution was dispensed into the corresponding wells. For the blank wells, 2.5 µL of Mab Anti 6HIS-XL665 (8 g/µL) diluted solution was added. Meanwhile, a reaction system was set up with a final compound concentration gradient ranging from 10 µM to 0.64 nM, KRAS (G12C) at 500 nM, MAb Anti GST-Eu cryptate at 0.25 ng/µL, SOS1 at 20 nM, and Mab Anti 6HIS-XL665 at 2 g/µL. This reaction system was incubated at 25°C for 60 min. After the reaction was completed, the HTRF was read using a multi-label analyzer.

### 4. Data analysis:

The raw data is converted into inhibition rates using the equation (Sample - **Min)/(Max - Min) × 100%.** The IC₅₀ value is then derived through a four-parameter curve fitting process (using the Log(inhibitor) vs. response -- Variable slope mode in GraphPad Prism). Table 12 presents the inhibitory activity of the compounds of the present disclosure against the binding of KRAS (G12C) and SOS1.

**Table 12 KRAS (G12C) and SOS1 binding inhibitory activity of compounds as shown in formula (I)**

| **Sample** | **IC₅₀(nM)** |
|---|---|
| Compounds as shown in formula (I) | 13 |

| | |
|---|---|
| [00244] Conclusion: the compound as shown in formula (I) has good KRAS (G12C)-SOS1 binding inhibitory activity. | |

### Experimental Embodiment 2. Experiment for detecting pERK expression levels in DLD-1 cells using HTRF

The phosphorylation level of ERK protein in DLD-1 cells with KRAS G13D mutation was detected by the Homogeneous Time-Resolved Fluorescence (HTRF) method, and the IC₅₀ and IC₉₀ values of compounds are employed as indicators to evaluate the inhibitory activity of these compounds towards ERK phosphorylation within the RAS signaling pathway.

### 1. Experimental procedures and methods:

1) DLD-1 cells (50,000 cells/well) were seeded into a 96-well plate, and each well was filled with 90 µL of RPMI-1640 medium (10% fetal bovine serum), and then placed in a 37°C, 5% carbon dioxide incubator for overnight culture.
2) 10 µL of 10X compound working solution were taken and added to the cell culture plate as shown in Table 13. 10 µL of DMSO-cell culture medium mixture were added to the solvent control and positive control, with a final DMSO concentration of 0.25%. The 96-well cell plate was then returned to the incubator for 1.5 h of culture.
3) 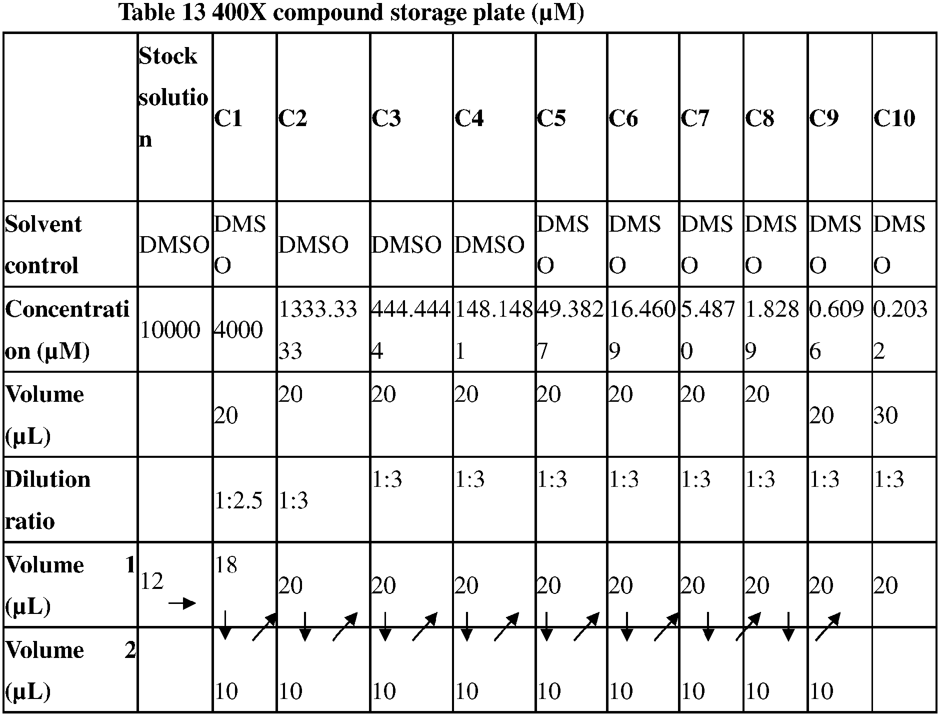
4) The cell supernatant was removed, and 50 µL of 1X lysis buffer was immediately added, followed by shaking and incubation at room temperature for 30 min.
5) A pipette was used to blow and mix the solution evenly, and 16 µL of cell lysate was transferred from the 96-well plate to the HTRF 384-well detection plate.
6) 4 µL of pre-mixed antibody solution were added, the plate was sealed, and incubated at 4°C. The fluorescence emission intensities at 665 nm and 620 nm were detected using an HTRF plate reader.

### 2. Data analysis:

1) Setting the fluorescence wavelengths of the Envision instrument: 665 nm and 620 nm, and performing fluorescence quantification;
2) Calculating the receptor and donor emission signals for each well: Ratio=Signal 665 nM/Signal 620 nM;
3) After subtracting background values from the raw data, the relative pERK levels were calculated by comparing them with the DMSO-treated group;
4) Using GraphPad 8.0 software to calculate IC₅₀ value based on Log[inhibitor] vs Response-variance slope. Table 14 presents the inhibitory activity of the compounds of the present disclosure against the pERK.

**Table 14. Inhibitory activity of compound as shown in formula (I) against p-ERK in DLD-1 cells**

| **Sample** | **IC₅₀(nM)** |
|---|---|
| Compounds as shown in formula (I) | 37 |

| | |
|---|---|
| Conclusion: the compound as shown in formula (I) exhibits significant inhibitory activity against the phosphorylation level of ERK kinase in DLD-1 cells. | |

### Experimental Embodiment 3. Anti-cell proliferation experiment of H358 cell 3D model

### Experimental materials:

RPMI1640 medium, fetal bovine serum, and penicillin/streptomycin antibiotics were purchased from Wisent, and low-melting agarose was purchased from Sigma. Almar blue reagent was purchased from Invitrogen. NCI-H358 cell line was purchased from Nanjing CoBioer Co., Ltd. Nivo multi-label analyzer (PerkinElmer).

### Experimental method:

H358 cells were seeded into a 96-well U-shaped plate. First, low-melting agarose was prepared into a 2% stock solution. Before use, the agarose stock solution was heated in a microwave oven to completely melt it, and then placed in a 42°C water bath to keep the agarose in a liquid state. The gel was added to the serum-containing medium to prepare a gel with a concentration of 0.6% as the bottom layer gel, which was then dispensed into the 96-well U-shaped plate at 50 µL per well. After the bottom layer gel solidifies, 2% gel was added to the cell-containing medium to prepare a cell-containing top layer gel with a gel concentration of 0.4%. The cell density was 4×10⁴ cells/mL, and 75 µL was added to each well of the 96-well U-shaped plate with the bottom layer gel, resulting in a cell density of 3000 cells per well. After the top layer gel solidifies, the cell plate was placed in a carbon dioxide incubator for overnight culture.

On the day of compound addition, 85 µL of liquid medium is added to the 96-well U-shaped plate with pre-seeded cells. The test compounds were subjected to a 3-fold dilution to the 9th concentration using a multi-channel pipette, ranging from 6 mM to 0.9 µM, and duplicate well experiment was set up for each concentration. 97 µL of medium was added to the intermediate plate, and then 2.5 µL of the gradient-diluted compound from each well was transferred to the corresponding position in the intermediate plate. After mixing, 40 µL of the mixture was transferred to each well of the cell plate. The concentration of compounds transferred into the cell plate ranged from 30 µM to 4.5 nM. The cell plate was then cultured in a carbon dioxide incubator for 7 days. On the 8th day, the test compounds were subjected to a 3-fold dilution to the 9th concentration using a multi-channel pipette, ranging from 6 mM to 0.9 µM, and duplicate well experiment was set up for each concentration. 198 µL of medium was added to the intermediate plate, and then 2 µL of the gradient-diluted compound from each well was transferred to the corresponding position in the first intermediate plate. Subsequently, 100 µL of medium was added to the second intermediate plate, and 100 µL of the mixed compound from the first intermediate plate is taken and added. After mixing, 40 µL of the mixture was transferred to each well of the cell plate. The concentration of compounds transferred into the cell plate ranged from 30 µM to 4.5 nM. The cell plate was placed in a carbon dioxide incubator and cultured for another 7 days. The compounds were co-incubated with the cells for 14 days. 20 µL of Almar blue detection reagent was added to each well of the cell plate. The plate with the dye was placed on a horizontal shaker and shaken for 15 min. Then, the plate was incubated at room temperature for 5 h to stabilize the luminescent signal. The readings were taken using a multi-label analyzer.

### Data analysis:

The raw data is converted into inhibition rates using the equation **(Sample - Min)/(Max - Min) × 100%.** The IC₅₀ value is then derived through a four-parameter curve fitting process (using the "log(inhibitor) vs. response -- Variable slope" mode in GraphPad Prism). Table 15 presents the inhibitory activity of the compounds of the present disclosure on H358 soft agar cell proliferation.

**Table 15. Anti-proliferation inhibitory activity of compound as shown in formula (I) in H358 cell 3D model**

| **Sample** | IC₅₀(**nM**) |
|---|---|
| Compounds as shown in formula (I) | 91 |

## Claims

1. A crystal form A of a compound as shown in formula (I) 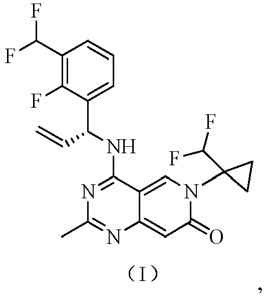 wherein an X-ray powder diffraction spectrum has diffraction peaks at the following 2θ angles: 9.41±0.20°, 19.23±0.20°, and 23.45±0.20°.

2. The crystal form A of the compound as shown in formula (I) according to claim 1, wherein the X-ray powder diffraction spectrum has diffraction peaks at the following 2θ angles: 9.41±0.20°, 13.39±0.20°, 13.96±0.20°, 15.77±0.20°, 19.23±0.20°, 21.99±0.20°, 23.45±0.20°, and 25.30±0.20°.

3. The crystal form A of the compound as shown in formula (I) according to claim 2, wherein the X-ray powder diffraction spectrum has diffraction peaks at the following 2θ angles: 9.41±0.20°, 12.03±0.20°, 12.70±0.20°, 13.39±0.20°, 13.96±0.20°, 15.77±0.20°, 19.23±0.20°, 21.99±0.20°, 23.45±0.20°, and 25.30±0.20°.

4. The crystal form A of the compound as shown in formula (I) according to claim 2, wherein the X-ray powder diffraction spectrum has diffraction peaks at the following 2θ angles: 9.41°, 10.13°, 12.03°, 12.70°, 13.39°, 13.96°, 14.67°, 14.98°, 15.77°, 16.12°, 16.67°, 17.94°, 18.28°, 18.86°, 19.23°, 19.69°, 20.12°, 21.99°, 22.35°, 22.74°, 23.45°, 23.71°, 24.35°, 25.30°, 25.80°, 26.15°, 26.51°, 28.38°, 29.94°, 30.34°, 30.64°, 32.02°, 32.87°, 37.62°, and 38.23°.

5. The crystal form A of the compound as shown in formula (I), wherein the X-ray powder diffraction spectrum is shown in FIG. 1.

6. The crystal form A of the compound as shown in formula (I) according to any one of claims 1 to 5, wherein a differential scanning calorimetry curve has an endothermic peak at 236.4±3°C.

7. The crystal form A of the compound as shown in formula (I) according to claim 6, wherein the differential scanning calorimetry curve spectrum is shown in FIG. 2.

8. The crystal form A of the compound as shown in formula (I) according to any one of claims 1 to 5, wherein a thermogravimetric analysis curve has a weight loss of 1.24% at 150.0±3°C.

9. The crystal form A of the compound as shown in formula (I) according to any one of claims 1 to 5, wherein the thermogravimetric analysis curve spectrum is shown in FIG. 3.

10. An application of the crystal form A of the compound as shown in formula (I) according to any one of claims 1 to 3 in a preparation of a treatment of lung cancer, pancreatic cancer or rectal cancer.
